# EUROPEAN PATENT APPLICATION

(11) **EP 4 686 481 A1**
(43) Date of publication of application: **04.02.2026**
(21) Application number: 24382853.0
(22) Date of filing: 31.07.2024
(51) Int. Cl.: A61K 48/00, C12N 15/11, C12N 15/64, C12N 15/79, C12P 19/34, C12Q 1/6806

(54) **DNA MOLECULES WITH METHYLATED AND UNMETHYLATED REGIONS**

(71) Applicant: 4basebio UK Ltd, Cambridge CB24 5QE (GB)
(72) Inventor: LANCKRIET, Heikki, Over, Cambridge CB24 5QE (GB); WALKER, Amy, Over, Cambridge CB24 5QE (GB); BOUCHAREB, Amine, Over, Cambridge CB24 5QE (GB); PAVLICKOVA, Milena, Over, Cambridge CB24 5QE (GB); MAHADEVA, Tejaswini, Over, Cambridge CB24 5QE (GB); PICHER, Ángel, E-28049 Cantoblanco, Madrid (ES)
(74) Representative: Boult Wade Tennant LLP

(57) **Abstract**

The invention relates to DNA molecules with a methylated region and an unmethylated region (e.g. with immunomodulatory properties), and uses thereof. The invention also relates to methods for producing DNA molecules and kits for use in such methods.

## Description

### TECHNICAL FIELD

The invention relates to DNA molecules with a methylated region and an unmethylated region (e.g. with immunomodulatory properties), and uses thereof. The invention also relates to methods for producing DNA molecules and kits for use in such methods.

### BACKGROUND

DNA is known to be immunostimulatory to eukaryotic cells. Whilst DNA from eukaryotic cells has methylated CpG motifs, prokaryotic DNA such as plasmid and synthetic DNA do not typically contain methylated CpG motifs. The Toll-like receptor 9 (TLR9) recognizes unmethylated CpG dinucleotides which are abundant in prokaryotic and or synthetic DNA and yet are rare in eukaryotic DNA. TLR9, preferentially binds DNA containing unmethylated CpGs, and triggers signalling cascades that lead to a pro-inflammatory cytokine response.

Gene therapies often require the introduction of DNA in eukaryotic cells. The main source of DNA is plasmid or synthetic DNA which are by default unmethylated. Whilst unmethylated DNA can be methylated for instance by use of methyltransferase enzymes or in case of synthetic DNA also by incorporation of methylated nucleotides in the synthesis process, lack of control over the methylation process results in the risk of methylating promoter regions thereby silencing the expression of the gene of interest.

Thus, a need exists for improved DNA molecules which have reduced immunostimulatory and/or toxic impact on eukaryotic cells whilst also providing improved expression levels.

### DESCRIPTION

### Methods for producing a DNA product

The invention provides a method for producing a deoxyribonucleic acid (DNA) product comprising an unmethylated region and a methylated region, wherein the method comprises appending a first DNA molecule comprising the unmethylated region to a second DNA molecule comprising the methylated region to generate the DNA product. Preferably, the DNA product is linear. By controlling which regions of the DNA product are unmethylated and methylated, the invention provides DNA products with altered immunomodulatory effects. For example, the DNA products may trigger lower levels of immunostimulation in eukaryotic cells. As a result, the DNA products are less vulnerable to silencing and, therefore, have improved expression levels in eukaryotic cells. Alternatively, the DNA products may trigger higher levels of immunostimulation in eukaryotic cells.

The invention provides a method for producing a deoxyribonucleic acid (DNA) product comprising an unmethylated region and a methylated region, wherein the methylated region comprises one or more methylated CpG, CpA, CpT and/or CpC sites, and wherein the method comprises:
appending a first DNA molecule comprising the unmethylated region to a second DNA molecule comprising the methylated region to generate the DNA product.

The invention provides a method for producing a deoxyribonucleic acid (DNA) product comprising an unmethylated region and a methylated region, wherein the unmethylated region comprises a promoter sequence, wherein the methylated region comprises one or more methylated CpG sites, and wherein the method comprises:
appending a first DNA molecule comprising the unmethylated region to a second DNA molecule comprising the methylated region to generate the DNA product.

The invention provides a method for producing a deoxyribonucleic acid (DNA) product comprising an unmethylated region and a methylated region, wherein the unmethylated region comprises a promoter sequence, wherein the methylated region comprises one or more methylated CpA sites, and wherein the method comprises:
appending a first DNA molecule comprising the unmethylated region to a second DNA molecule comprising the methylated region to generate the DNA product.

The invention also provides a method for producing a deoxyribonucleic acid (DNA) product comprising an unmethylated region and a methylated region, wherein the unmethylated region comprises a promoter sequence, wherein the methylated region comprises one or more methylated CpG sites and wherein the methylated region comprises a coding sequence encoding a gene of interest, and wherein the method comprises:
appending a first DNA molecule comprising the unmethylated region to a second DNA molecule comprising the methylated region to generate the DNA product.

The invention also provides a method for producing a deoxyribonucleic acid (DNA) product comprising an unmethylated region and a methylated region, wherein the unmethylated region comprises a promoter sequence, wherein the methylated region comprises one or more methylated CpA sites and wherein the methylated region comprises a coding sequence encoding a gene of interest, and wherein the method comprises:
appending a first DNA molecule comprising the unmethylated region to a second DNA molecule comprising the methylated region to generate the DNA product.

The invention also provides a method for producing a deoxyribonucleic acid (DNA) product comprising an unmethylated region and a methylated region, wherein the unmethylated region comprises a promoter sequence, wherein the methylated region comprises one or more methylated CpG sites and wherein the methylated region comprises a coding sequence encoding a gene of interest, and wherein the method comprises:
appending a first DNA molecule comprising the unmethylated region to a second DNA molecule comprising the methylated region to generate the DNA product, wherein the promoter sequence is capable of initiating transcription of the coding sequence.

The invention also provides a method for producing a deoxyribonucleic acid (DNA) product comprising an unmethylated region and a methylated region, wherein the unmethylated region comprises a promoter sequence, wherein the methylated region comprises one or more methylated CpA sites and wherein the methylated region comprises a coding sequence encoding a gene of interest, and wherein the method comprises:
appending a first DNA molecule comprising the unmethylated region to a second DNA molecule comprising the methylated region to generate the DNA product, wherein the promoter sequence is capable of initiating transcription of the coding sequence.

In the methods, the step of appending the first DNA molecule comprising the unmethylated region to the second DNA molecule comprising the methylated region may be performed by ligating the first DNA molecule to the second DNA molecule.

The method may comprise amplifying a first DNA template molecule to generate the first DNA molecule comprising the unmethylated region. Thus, the first DNA molecule may be generated by amplifying a first DNA template molecule. Alternatively, the first DNA molecule may be a synthetic DNA molecule. The method may comprise synthesising the first DNA molecule by solid-phase synthesis.

The method may comprise amplifying a second DNA template molecule in the presence of methylated nucleotide triphosphates to generate the second DNA molecule comprising the methylated region. Thus, the second DNA molecule may be generated by amplifying a second DNA template molecule in the presence of methylated nucleotide triphosphates.

The method may comprise contacting a precursor DNA molecule with a methyltransferase to generate the second DNA molecule comprising the methylated region; optionally wherein the precursor DNA molecule is substantially free from methylated nucleotides. Thus, the second DNA molecule may be generated by contacting a precursor DNA molecule with a methyltransferase.

The method may comprise amplifying a second DNA template molecule to generate the precursor DNA molecule. Alternatively, the precursor DNA molecule may be a synthetic DNA molecule. The method may comprise synthesising the precursor DNA molecule by solid-phase synthesis.

The method may comprise inactivating or removing the methyltransferase prior to appending the first DNA molecule to the second DNA molecule.

The DNA product may be linear and the method may further comprise:
(a) appending a first adaptor molecule to a first end of the DNA product and appending a second adaptor molecule to a second end of the DNA product, wherein the first adaptor molecule comprises a hairpin and/or one or more nuclease resistant nucleotides and the second adaptor molecule comprises a hairpin and/or one or more nuclease resistant nucleotides; or
(b) covalently closing one or both ends of the DNA product (e.g. using a protelomerase).

Appending the first DNA molecule to the second DNA molecule may comprise:
(a) contacting the first DNA molecule with an endonuclease, a ligase and the second DNA molecule to form a single contiguous aqueous volume; and
(b) incubating the single contiguous aqueous volume to generate the DNA product.

Appending the first DNA molecule to the second DNA molecule may comprise:
(i) contacting the first DNA molecule and second DNA molecule with an endonuclease to form a digested first DNA molecule and a digested second DNA molecule;
(ii) contacting the digested first DNA molecule and the digested second DNA molecule with a ligase to generate the DNA product.

The unmethylated region may be 5' to the methylated region in the DNA product. The unmethylated region may comprise CpG sites. The unmethylated region may comprise complementary sense and antisense strands. The methylated region may comprise a coding sequence encoding a gene of interest. The methylated region may comprise complementary sense and antisense strands.

The DNA product may be a double-stranded DNA molecule, a single-stranded DNA molecule or it may be a double-stranded DNA molecule with one or more single-stranded regions.

The DNA product may be linear and comprise a closed loop and/or one or more nuclease-resistant nucleotides at one or both ends.

The DNA product may consist of the unmethylated region and the methylated region. The first DNA molecule may consist of the unmethylated region. The second DNA molecule may consist of the methylated region.

The first DNA molecule may be substantially free from methylated CpG, CpA, CpT and/or CpC sites. Thus, no more than 10%, no more than 5%, no more than 1% or 0% of CpG sites may be methylated in the first DNA molecule. Preferably, no more than 5% of CpG, CpA, CpT and/or CpC sites are methylated in the first DNA molecule.

The second DNA molecule may be methylated on substantially all CpG, CpA, CpT and/or CpC sites. Thus, at least 50%, at least 75%, at least 90%, at least 95%, at least 99% or 100% of CpG, CpA, CpT and/or CpC sites may be methylated in the methylated region. Preferably, at least 90% of CpG, CpA, CpT and/or CpC sites are methylated in the second DNA molecule.

The first DNA molecule may be substantially free from methylated CpG sites. Thus, no more than 10%, no more than 5%, no more than 1% or 0% of CpG sites may be methylated in the first DNA molecule. Preferably, no more than 5% of CpG sites are methylated in the first DNA molecule.

The second DNA molecule may be methylated on substantially all CpG sites. Thus, at least 50%, at least 75%, at least 90%, at least 95%, at least 99% or 100% of CpG sites may be methylated in the methylated region. Preferably, at least 90% of CpG sites are methylated in the second DNA molecule.

The first DNA molecule may be substantially free from methylated CpA sites. Thus, no more than 10%, no more than 5%, no more than 1% or 0% of CpG sites may be methylated in the first DNA molecule. Preferably, no more than 5% of CpA sites are methylated in the first DNA molecule.

The second DNA molecule may be methylated on substantially all CpA sites. Thus, at least 50%, at least 75%, at least 90%, at least 95%, at least 99% or 100% of CpA sites may be methylated in the methylated region. Preferably, at least 90% of CpA sites are methylated in the second DNA molecule.

The unmethylated region may be free from methylation on substantially all CpG, CpA, CpT and/or CpC sites. In other words, the unmethylated region may not be methylated on substantially all CpA sites. The unmethylated region may be substantially free from methylated CpA sites. Thus, no more than 10%, no more than 5%, no more than 1% or 0% of CpA sites may be methylated in the unmethylated region. Preferably, no more than 5% of CpA sites are methylated in the unmethylated region.

The unmethylated region may be free from methylation on substantially all CpG sites. In other words, the unmethylated region may not be methylated on substantially all CpG sites. The unmethylated region may be substantially free from methylated CpG sites. Thus, no more than 10%, no more than 5%, no more than 1% or 0% of CpG sites may be methylated in the unmethylated region. Preferably, no more than 5% of CpG sites are methylated in the unmethylated region.

The unmethylated region may comprise methylated adenine (i.e. N6-Methyladenine). The unmethylated region may comprise 4-Methylcytosine (4mC).

The unmethylated region may be free from methylation on substantially all CpA sites. In other words, the unmethylated region may not be methylated on substantially all CpA sites. The unmethylated region may be substantially free from methylated CpA sites. Thus, no more than 10%, no more than 5%, no more than 1% or 0% of CpA sites may be methylated in the unmethylated region. Preferably, no more than 5% of CpA sites are methylated in the unmethylated region.

The unmethylated region may comprise a promoter sequence. A promoter is a region of DNA to which proteins (such as RNA polymerase and transcription factors) bind to initiate transcription upstream of the promoter. A promoter sequence may comprise the sequence of a promoter or portion thereof. The promoter sequence may comprise a first promoter sequence which forms a functional promoter sequence only when a second promoter sequence is appended thereto. The second promoter sequence may be provided by the methylated region or an adapter. Thus, when the methylated region or an adapter is appended to the first promoter sequence in the unmethylated region a functional promoter sequence may form from which transcription can be initiated.

The promoter may promote transcription in a eukaryotic cell, preferably a mammalian cell. The promoter may comprise CpG, CpA, CpT and/or CpC sites.

The promoter may promote transcription in a eukaryotic cell, preferably a mammalian cell. The promoter may comprise CpG sites.

The promoter may be a CMV promoter, CAG promoter, EF1α promoter, PGK promoter, MHCK7 promoter, SV40 promoter, RSV promoter and/or UBC promoter.

The unmethylated region may comprise a regulatory sequence. The regulatory sequence may comprise: an enhancer and a promoter; a promoter and a 5'UTR; or an enhancer, a promoter and a 5'UTR.

The methylated region may be methylated on substantially all CpG sites. Substantially all CpG sites in the methylated region may be methylated. Thus, at least 50%, at least 75%, at least 80%, at least 85%, at least 90%, at least 95%, at least 99% or 100% of CpG sites may be methylated in the methylated region. Preferably, at least 85% of CpG sites are methylated in the methylated region. Preferably, the methylated cytosine in CpG sites is 5-Methylcytosine (5mC).

The methylated region may be methylated at non-CpG sites e.g. CpA, CpT and/or CpC sites.

The methylated region may comprise a coding sequence or an open reading frame. The coding sequence may encode a gene of interest, for example a gene encoding a protein. The gene may comprise at least 200, at least 500, at least 1000, least 1500, at least 2000, at least 3000, at least 4000, at least 5000, at least 7500, at least 10,000 nucleotides. Preferably, the gene comprises at least 200 nucleotides. The open reading frame may comprise at least 200, at least 500, at least 1000, least 1500, at least 2000, at least 3000, at least 4000, at least 5000, at least 7500, at least 10,000, at least 15,000 or at least 20,000 nucleotides. Preferably, the open reading frame comprises at least 200 nucleotides.

The methylated region may comprise (or encode) a repair template (or editing template). The repair template (or editing template) may be for use in CRISPR-Cas mediated homology directed repair (HDR). The second region may encode CRISPR guide RNA.

The methylated region may comprise a cassette. The methylated region may comprise a single cassette. The term "single cassette" as used herein is intended to encompass a product that does not comprise or consist of a plurality of cassettes. That is to say that the methylated region may comprise only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The cassette may comprise a coding sequence or an open reading frame. The coding sequence may encode a gene of interest, for example a gene encoding a protein. The gene may comprise at least 200, at least 500, at least 1000, least 1500, at least 2000, at least 3000, at least 4000, at least 5000, at least 7500, at least 10,000 nucleotides. Preferably, the gene comprises at least 200 nucleotides. The open reading frame may comprise at least 200, at least 500, at least 1000, least 1500, at least 2000, at least 3000, at least 4000, at least 5000, at least 7500, at least 10,000 nucleotides. Preferably, the open reading frame comprises at least 200 nucleotides.

The cassette may comprise at least a portion of a promoter and a coding sequence or open reading frame. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The ssDNA cassette may encode CRISPR guide RNA. The cassette may be a mammalian expression cassette. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

A CpG site is a region in a DNA sequence, which comprises cytosine followed by guanine in the 5' to 3' direction. A methylated CpG site or a CpG site that is methylated is a CpG site in which the cytosine is methylcytosine, preferably 5- methylcytosine (5mC).

A CpA site is a region in a DNA sequence, which comprises cytosine followed by adenine in the 5' to 3' direction. A methylated CpA site or a CpA site that is methylated is a CpA site in which the cytosine is methylcytosine, preferably 5- methylcytosine (5mC).

A CpT site is a region in a DNA sequence, which comprises cytosine followed by thymine in the 5' to 3' direction. A methylated CpT site or a CpT site that is methylated is a CpT site in which the cytosine is methylcytosine, preferably 5- methylcytosine (5mC).

A CpT site is a region in a DNA sequence, which comprises cytosine followed by another cytosine in the 5' to 3' direction. A methylated CpC site or a CpC site that is methylated is a CpC site in which the first cytosine is methylcytosine, preferably 5- methylcytosine (5mC).

Appending the first DNA molecule to the second DNA molecule may be achieved by any means known in the art. For examples, see the following references which are incorporated herein by reference: Chao et al. Recent advances in DNA assembly technologies. FEMS Yeast Res. 2015 Feb;15(1):1-9. doi: 10.1111/1567-1364.12171; and Baek et al. DNA Assembly Tools and Strategies for the Generation of Plasmids. Microbiol Spectr. 2014 Oct;2(5). doi: 10.1128/microbiolspec.PLAS-0014-2013.

These means include, but are not limited to, endonuclease-based cloning, recombination-based cloning, topoisomerase-based cloning, isothermal assembly reaction (Gibson Assembly), ligation independent cloning and overlap extension PCR.

Appending may refer to covalently linking one molecule to another. Thus, appending the first DNA molecule to the second DNA molecule may involve covalently linking the first DNA molecule to the second DNA molecule. In the DNA product, the first DNA molecule may be covalently linked to the second DNA molecule. Thus, in the DNA product there may be a covalent linkage between the first DNA molecule and second DNA molecule.

The first DNA molecule may be appended to the second DNA molecule using a ligase, recombinase, topoisomerase or polymerase. Preferably, the first DNA molecule is appended to the second DNA molecule by a ligase. The first and/or second DNA molecules may undergo processing before being appended together (e.g. undergo endonuclease digestion).

The ligase may be a DNA ligase, such as a T3 DNA ligase, a T4 DNA ligase, T7 DNA ligase, mammalian DNA ligase I, III and IV; Taq DNA ligase, Tth DNA ligase, and/or E. *coli* DNA ligase.

The first and second DNA molecules may each comprise at least one endonuclease target site which after digestion by an endonuclease generates digested first and second DNA molecules which may be ligated together by a ligase.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQl, BtgZl, Esp3I,SapI, Aarl, Acc36I, AcIWI, Acul, Ajul, AloI, Alw26I, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3l, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI restriction enzyme.

Type IIS restriction endonucleases cleave double-stranded DNA outside of the recognition sequence (i.e. an endonuclease target sequence), which means that the recognition sequence (i.e. endonuclease target sequence) is not included in the DNA product.

The first and second DNA molecules (and optionally also the first and second DNA template molecules) may each comprise an endonuclease target sequence. The endonuclease target sequence may be a restriction endonuclease target sequence. Different restriction endonuclease target sequences would be known to the skilled person. The endonuclease target sequence may be a Type IIS restriction endonuclease target sequence. For example, the restriction endonuclease target sequence may be a Bbsl, Bsal, BsmBI, BspQl, BtgZl, Esp3I,SapI, Aarl, Acc36I, AcIWI, Acul, Ajul, AloI, AIw26I, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, BsuI, BtgZI, BtsCl, Btsl-v2, BtsMutl, Bvel, Csel, CspCI, Eam1104I, Earl, Ecil, Eco31I, Eco57I, Esp3l, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI target sequence.

Amplification may be performed by any means known in the art including, but not limited to, rolling circle amplification (RCA), multiple annealing and looping-based amplification cycles (MALBAC) method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. Rolling circle amplification may be performed *in vitro* under isothermal conditions using a suitable nucleic acid polymerase, such as Phi29 DNA polymerase.

Amplifying the first DNA template molecule may be performed using the same or a different amplification method to amplifying the second DNA template molecule.

The methylated nucleotide triphosphates may comprise 5-methyl-dCTP (2'-deoxy-5-methylcytidine 5'-tri phosphate).

The first DNA template molecule may comprise a promoter sequence as described herein. The first DNA template may comprise a promoter sequence as described herein. The digested first DNA template may comprise a promoter sequence as described herein.

The second DNA template molecule may comprise a coding sequence as described herein. The precursor DNA molecule may comprise a coding sequence as described herein. The second DNA molecule may comprise a coding sequence as described herein. The digested second DNA molecule may comprise a coding sequence as described herein.

The methyltransferase may be a methyltransferase which transfers a methyl group from a methyl donor (such as S-adenosylmethionine) to cytosine. Contacting the precursor DNA molecule with a methyltransferase thus includes contacting the precursor DNA molecule with the methyltransferase in the presence of a methyl donor (such as S-adenosylmethionine). Preferably, the precursor DNA molecule is contacted with a methyltransferase in the presence of S-adenosylmethionine.

The methyltransferase may be a DNA methyltransferase. The methyltransferase may be a m4C or m5C methyltransferase. An m4C methyltransferase (N-4 cytosine-specific DNA methylases) specifically methylates the amino group at the C-4 position of cytosines in DNA. An m5C methyltransferase (C-5 cytosine-specific DNA methylase) (C5 Mtase) specifically methylates the C-5 carbon of cytosines in DNA to produce C5-methylcytosine. Preferably, the methyltransferase is an m5C methyltransferase

Methyltransferases include, but are not limited to, CpG Methyltransferase (M.Sssl), Hhal Methyltransferase and Hpall Methyltransferase.

The methyltransferase may be incubated with the second DNA molecule at 37°C for at least 1 hour. The methyltransferase may be deactivated by heating (for example 65°C for no more or at least 20 minutes). The methyltransferase may be separated from the second DNA molecule by any means known in the art (for example, the methyltransferase may comprise an affinity tag).

The step of appending a first adaptor molecule to a first end of the DNA product and appending a second adaptor molecule to a second end of the DNA product may be performed as described in WO2023/006978 which is hereby incorporated by reference.

The DNA product may comprise at least one endonuclease target sequence as described herein. The DNA product may comprise at least one endonuclease target sequence 5' of the unmethylated region and/or at least one endonuclease target sequence 3' of the methylated region.

The methods may further comprise contacting the DNA product with an endonuclease and first and second adaptor molecules to form a single contiguous aqueous volume; and incubating the single contiguous aqueous volume to generate a DNA product (i.e. an adapter appended DNA product), wherein the first adaptor molecule is appended to a first end of the DNA product (i.e. 5' of the unmethylated region) and the second adaptor molecule is appended to a second end of the DNA product (i.e. 3' of the methylated region).

The methods may further comprise contacting the DNA product with an endonuclease, a ligase and first and second adaptor molecules to form a single contiguous aqueous volume, wherein the first and second adaptor molecules comprise a closed loop; and incubating the single contiguous aqueous volume to generate a closed DNA product, wherein the closed DNA product is closed at a first end by the first adaptor molecule and closed at a second end by the second adaptor molecule.

The first and second adaptor molecules may be identical molecules, or they may be different molecules. For example, the first adaptor molecule and/or the second adaptor molecule may comprise a hairpin or be linear. The first adaptor molecule and/or the second adaptor molecule may comprise one or more nuclease-resistant nucleotides. The first adaptor molecule may comprise a hairpin and the second adaptor molecule may be linear and comprise one or more nuclease-resistant nucleotides. The second adaptor molecule may comprise a hairpin and the first adaptor molecule may be linear and comprise one or more nuclease-resistant nucleotides. Thus, the DNA product produced by the methods described herein may be resistant to nuclease (e.g. exonuclease) digestion.

The first adaptor molecule and/or the second adaptor molecule may be nucleic acid adaptor molecules. The first adaptor molecule may comprise a hairpin and/or the second adaptor molecule may comprise a hairpin. The first adaptor molecule and/or the second adaptor molecule may comprise a double-stranded region with an overhang. The adapter may comprise one or more nuclease-resistant nucleotides such as phosphorothioated nucleotides.

As used herein the term "nuclease-resistant nucleotide" is intended to encompass any type of molecule that provides or enhances resistance to nuclease digestion (especially exonuclease digestion).

Nuclease-resistant nucleotides may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nuclease-resistant nucleotides may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The DNA product may comprise a protelomerase target sequence at one or both ends. The DNA product comprising a protelomerase target sequence at one or both ends may be incubated with a protelomerase to generate a closed end from each protelomerase target sequence. Thus, the DNA product may be closed at one or both ends.

The DNA product may comprise a truncated protelomerase sequence at one or both ends. A functional protelomerase target sequence may be formed at one or both ends of the DNA product by appending to one or both ends an adaptor molecule comprising the rest of the protelomerase target sequence. Appending an adaptor molecule to the DNA product may be performed by any means of appending as described herein.

The DNA product comprising a truncated protelomerase sequence at one or both ends may be generated by digesting the DNA product with one or more endonucleases that cleave one or more endonuclease target sequences in the DNA product to generate a digested DNA molecule with a truncated protelomerase sequence at one or both ends.

As used herein, "truncated protelomerase target sequence" refers to a protelomerase target sequence that has had sufficient base pairs removed such that protelomerase no longer processes the DNA to produce closed ends, i.e., the protelomerase is unable to cleave and re-ligate the DNA to form covalently closed DNA. In other words, the truncated protelomerase sequence as used herein refers to a non-functional protelomerase sequence.

The protelomerase may be TelN protelomerase from E. *coli* phage N15 TelN protelomerase along with the sequence defined herein. Alternatively, the protelomerase may be Klebsiella phage Phi K02 protelomerase, Yersinia phage PY54 protelomerase, Vibrio phage VP882 protelomerase, Borrelia burgdorferi protelomerase.

If a protelomerase other than TelN is used, the truncated protelomerase target sequence may be a truncated variant of a protelomerase target sequence specific for the protelomerase enzyme that is used.

A closed DNA product has particular utility as a therapeutic agent (i.e. DNA therapeutic) which can be used to express a gene product *in vivo.* This is because its closed structure (e.g. covalently closed structure) prevents attack by enzymes such as exonucleases, leading to enhanced stability and longevity of gene expression as compared to "open" DNA molecules with exposed DNA ends.

Sequestering DNA ends inside closed structures also has other advantages. The DNA ends are prevented from integrating with genomic DNA and so closed linear DNA products offer improved safety. In addition, the closed linear structure reduces concatamerisation of DNA products inside host cells and thus expression levels of the gene product can be regulated in a more sensitive manner.

The first DNA molecule may be amplified from a first DNA template molecule and/or the second DNA molecule may be amplified from a second DNA template molecule.

The DNA product may comprise a linear portion of the first DNA molecule and a linear portion of the second DNA molecule. The method of the invention produces the DNA product (e.g. the linear DNA product) in a single reaction volume (or single contiguous aqueous volume) comprising a first DNA molecule, a second DNA molecule, an endonuclease and a ligase. Thus, the method for producing a DNA product (e.g. the linear DNA product) comprises: a) forming a single contiguous aqueous volume comprising a first DNA molecule, a second DNA molecule, an endonuclease and a ligase; and b) incubating the single contiguous aqueous volume to generate the DNA product (e.g. the linear DNA product), wherein the DNA product (e.g. the linear DNA product) comprises a linear double-stranded region, wherein the linear double-stranded region comprises a linear portion of the first DNA molecule and a linear portion of the second DNA molecule. Preferably, the first DNA molecule and/or the second DNA molecule is/are product(s) of rolling circle amplification (RCA). Preferably, the first DNA molecule is a first concatemer and/or the second DNA molecule is a second concatemer.

The invention provides a method for producing a linear deoxyribonucleic acid (DNA) product, wherein the method comprises:
a) amplifying a first DNA template molecule to generate a first DNA molecule, wherein the first DNA template molecule comprises at least one cleavable target sequence (e.g. endonuclease target sequence), and amplifying a second DNA template molecule to generate a second DNA molecule, wherein the second DNA template molecule comprises at least one cleavable target sequence (e.g. endonuclease target sequence);
b) forming a single contiguous aqueous volume comprising the first DNA molecule, the second DNA molecule, an endonuclease and a ligase; and
c) incubating the single contiguous aqueous volume to generate the DNA product (e.g. the linear DNA product), wherein the DNA product (e.g. the linear DNA product) comprises a linear double-stranded region, wherein the linear double-stranded region comprises a linear portion of the first DNA molecule and a linear portion of the second DNA molecule.

The invention provides a method for producing a linear deoxyribonucleic acid (DNA) product, wherein the method comprises:
a) amplifying a first DNA template molecule to generate a first DNA molecule, wherein the first DNA template molecule comprises at least one cleavable target sequence (e.g. endonuclease target sequence) and wherein the first DNA molecule is a first concatemer, and amplifying a second DNA template molecule to generate a second DNA molecule, wherein the second DNA template molecule comprises at least one cleavable target sequence (e.g. endonuclease target sequence) and wherein the second DNA molecule is a second concatemer;
b) forming a single contiguous aqueous volume comprising the first DNA molecule, the second DNA molecule, an endonuclease and a ligase; and
c) incubating the single contiguous aqueous volume to generate the DNA product (e.g. the linear DNA product), wherein the DNA product (e.g. the linear DNA product) comprises a linear double-stranded region, wherein the linear double-stranded region comprises a linear portion of the first DNA molecule and a linear portion of the second DNA molecule.

The invention provides a method for producing a linear deoxyribonucleic acid (DNA) product, wherein the method comprises:
a) amplifying a first DNA template molecule by rolling circle amplification to generate a first DNA molecule, wherein the first DNA template molecule comprises at least one cleavable target sequence (e.g. endonuclease target sequence) and wherein the first DNA molecule is a first concatemer, and amplifying a second DNA template molecule by rolling circle amplification to generate a second DNA molecule, wherein the second DNA template molecule comprises at least one cleavable target sequence (e.g. endonuclease target sequence) and wherein the second DNA molecule is a second concatemer;
b) forming a single contiguous aqueous volume comprising the first DNA molecule, the second DNA molecule, an endonuclease and a ligase; and
c) incubating the single contiguous aqueous volume to generate the DNA product (e.g. the linear DNA product), wherein the DNA product (e.g. the linear DNA product) comprises a linear double-stranded region, wherein the linear double-stranded region comprises a linear portion of the first DNA molecule and a linear portion of the second DNA molecule.

In the methods, the step of incubating the single contiguous aqueous volume to generate the DNA product (e.g. the linear DNA product) may comprise:
i) digesting the first DNA molecule with the endonuclease by cleaving the cleavable target sequence (e.g. endonuclease target sequence) to generate the linear portion of the first DNA molecule;
ii) digesting the second DNA molecule with the endonuclease by cleaving the cleavable target sequence (e.g. endonuclease target sequence) to generate the linear portion of the second DNA molecule; and
iii) ligating the linear portion of the first DNA molecule to the linear portion of the second DNA molecule to generate the linear double-stranded region.

The linear DNA product may be an open linear DNA product, optionally wherein the linear DNA product comprises a first adaptor molecule at a first end and a second adaptor molecule at a second end.

The linear DNA product may be a closed linear DNA product, optionally wherein the linear double-stranded region is closed at a first end by a first adaptor molecule and closed at a second end by a second adaptor molecule. The linear double-stranded region may be closed at a first end by protelomerase (e.g. TeIN) or closed at a second end by protelomerase (e.g. TeIN), optionally wherein a first adaptor molecule comprises a protelomerase target sequence or a portion thereof and/or a second adaptor molecule comprises a protelomerase target sequence or a portion thereof. The linear double-stranded region may be closed at a first end by a first adaptor molecule and closed at a second end by protelomerase (e.g. TeIN) (optionally wherein a second adaptor molecule comprises a protelomerase target sequence or a portion thereof). The linear double-stranded region may be closed at a first end by protelomerase (optionally wherein a first adaptor molecule comprises a protelomerase target sequence or a portion thereof) and closed at a second end by a second adaptor molecule.

The linear double-stranded region may be a partially closed linear DNA, optionally wherein the linear double-stranded region is closed at a first end by a first adaptor molecule or closed at a second end by a second adaptor molecule. The partially closed linear double-stranded region may be closed at a first end by protelomerase (e.g. TeIN), optionally wherein a first adaptor molecule comprises a protelomerase target sequence or a portion thereof. The partially closed linear double-stranded region may be closed at a second end by protelomerase (e.g. TeIN), optionally wherein a second adaptor molecule comprises a protelomerase target sequence or a portion thereof.

The invention provides a method for producing a DNA product (e.g. the linear DNA product), wherein the method comprises:
a) amplifying a first DNA template molecule (e.g. by rolling circle amplification) to generate a first DNA molecule, wherein the first DNA template molecule comprises at least one cleavable target sequence (e.g. endonuclease target sequence), and amplifying a second DNA template molecule (e.g. by rolling circle amplification) to generate a second DNA molecule, wherein the second DNA template molecule comprises at least one cleavable target sequence (e.g. endonuclease target sequence);
b) forming a single contiguous aqueous volume comprising the first DNA molecule, the second DNA molecule, an endonuclease, a ligase, and first and second adaptor molecules; and
c) incubating the single contiguous aqueous volume to generate the DNA product (e.g. the linear DNA product), wherein the DNA product comprises a linear double-stranded region, wherein the linear double-stranded region comprises a linear portion of the first DNA molecule and a linear portion of the second DNA molecule, and wherein the first adaptor molecule is ligated to a first end of the linear double-stranded region and the second adaptor molecule is ligated to a second end of the linear double-stranded region.

The invention provides a method for producing a DNA product (e.g. the linear DNA product), wherein the method comprises:
a) amplifying a first DNA template molecule (e.g. by rolling circle amplification) to generate a first DNA molecule, wherein the first DNA template molecule comprises at least one cleavable target sequence (e.g. endonuclease target sequence) and wherein the first DNA molecule is a first concatemer, and amplifying a second DNA template molecule (e.g. by rolling circle amplification) to generate a second DNA molecule, wherein the second DNA template molecule comprises at least one cleavable target sequence (e.g. endonuclease target sequence) and wherein the second DNA molecule is a second concatemer;
b) forming a single contiguous aqueous volume comprising the first DNA molecule, the second DNA molecule, an endonuclease, a ligase, and first and second adaptor molecules; and
c) incubating the single contiguous aqueous volume to generate the DNA product (e.g. the linear DNA product), wherein the DNA product comprises a linear double-stranded region, wherein the linear double-stranded region comprises a linear portion of the first DNA molecule and a linear portion of the second DNA molecule, and wherein the first adaptor molecule is ligated to a first end of the linear double-stranded region and the second adaptor molecule is ligated to a second end of the linear double-stranded region.

In the methods, the step of incubating the single contiguous aqueous volume to generate the DNA product (e.g. the linear DNA product) may comprise:
i) digesting the first DNA molecule with the endonuclease by cleaving the cleavable target sequence (e.g. endonuclease target sequence) to generate the linear portion of the first DNA molecule;
ii) digesting the second DNA molecule with the endonuclease by cleaving the cleavable target sequence (e.g. endonuclease target sequence) to generate the linear portion of the second DNA molecule; and
iii) ligating the first adaptor molecule to a first end of the linear portion of the first DNA molecule, ligating the second end of the linear portion of the first DNA molecule to the first end of the linear portion of the second DNA molecule and ligating the second end of the linear portion of the second DNA molecule to the second adaptor molecule.

The invention provides a method for producing a closed linear DNA product, wherein the method comprises:
a) amplifying a first DNA template molecule (e.g. by rolling circle amplification) to generate a first DNA molecule, wherein the first DNA template molecule comprises at least one cleavable target sequence (e.g. endonuclease target sequence), and amplifying a second DNA template molecule (e.g. by rolling circle amplification) to generate a second DNA molecule, wherein the second DNA template molecule comprises at least one cleavable target sequence (e.g. endonuclease target sequence);
b) forming a single contiguous aqueous volume comprising the first DNA molecule, the second DNA molecule, an endonuclease, a ligase, and first and second adaptor molecules; and
c) incubating the single contiguous aqueous volume to generate the closed linear DNA product, wherein the closed linear DNA product comprises a linear double-stranded region, wherein the linear double-stranded region comprises a linear portion of the first DNA molecule and a linear portion of the second DNA molecule, and wherein the linear double-stranded region is closed at a first end by the first adaptor molecule and closed at a second end by the second adaptor molecule.

The invention provides a method for producing a closed linear DNA product, wherein the method comprises:
a) amplifying a first DNA template molecule (e.g. by rolling circle amplification) to generate a first DNA molecule, wherein the first DNA template molecule comprises at least one cleavable target sequence (e.g. endonuclease target sequence) and wherein the first DNA molecule is a first concatemer, and amplifying a second DNA template molecule (e.g. by rolling circle amplification) to generate a second DNA molecule, wherein the second DNA template molecule comprises at least one cleavable target sequence (e.g. endonuclease target sequence) and wherein the second DNA molecule is a second concatemer;
b) forming a single contiguous aqueous volume comprising the first DNA molecule, the second DNA molecule, an endonuclease, a ligase, and first and second adaptor molecules; and
c) incubating the single contiguous aqueous volume to generate the closed linear DNA product, wherein the closed linear DNA product comprises a linear double-stranded region, wherein the linear double-stranded region comprises a linear portion of the first DNA molecule and a linear portion of the second DNA molecule, and wherein the linear double-stranded region is closed at a first end by the first adaptor molecule and closed at a second end by the second adaptor molecule.

In the methods, the step of incubating the single contiguous aqueous volume to generate the closed linear DNA product may comprise:
i) digesting the first DNA molecule with the endonuclease by cleaving the cleavable target sequence (e.g. endonuclease target sequence) to generate the linear portion of the first DNA molecule;
ii) digesting the second DNA molecule with the endonuclease by cleaving the cleavable target sequence (e.g. endonuclease target sequence) to generate the linear portion of the second DNA molecule; and
iii) ligating the first adaptor molecule to a first end of the linear portion of the first DNA molecule, ligating the second end of the linear portion of the first DNA molecule to the first end of the linear portion of the second DNA molecule and ligating the second end of the linear portion of the second DNA molecule to the second adaptor molecule.

The invention provides a method for producing an open linear DNA product, wherein the method comprises:
a) amplifying a first DNA template molecule (e.g. by rolling circle amplification) to generate a first DNA molecule, wherein the first DNA template molecule comprises at least one cleavable target sequence (e.g. endonuclease target sequence), and amplifying a second DNA template molecule (e.g. by rolling circle amplification) to generate a second DNA molecule, wherein the second DNA template molecule comprises at least one cleavable target sequence (e.g. endonuclease target sequence);
b) forming a single contiguous aqueous volume comprising the first DNA molecule, the second DNA molecule, an endonuclease, a ligase, and first and second adaptor molecules; and
c) incubating the single contiguous aqueous volume to generate the open linear DNA product, wherein the open linear DNA product comprises a linear double-stranded region, wherein the linear double-stranded region comprises a linear portion of the first DNA molecule and a linear portion of the second DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules (e.g. DNA molecules) that each comprise one or more nuclease-resistant nucleotides.

The invention provides a method for producing an open linear DNA product, wherein the method comprises:
a) amplifying a first DNA template molecule (e.g. by rolling circle amplification) to generate a first DNA molecule, wherein the first DNA template molecule comprises at least one cleavable target sequence (e.g. endonuclease target sequence) and wherein the first DNA molecule is a first concatemer, and amplifying a second DNA template molecule (e.g. by rolling circle amplification) to generate a second DNA molecule, wherein the second DNA template molecule comprises at least one cleavable target sequence (e.g. endonuclease target sequence) and wherein the second DNA molecule is a second concatemer;
b) forming a single contiguous aqueous volume comprising the first DNA molecule, the second DNA molecule, an endonuclease, a ligase, and first and second adaptor molecules; and
c) incubating the single contiguous aqueous volume to generate the open linear DNA product, wherein the open linear DNA product comprises a linear double-stranded region, wherein the linear double-stranded region comprises a linear portion of the first DNA molecule and a linear portion of the second DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules (e.g. DNA molecules) that each comprise one or more nuclease-resistant nucleotides.

In the methods, the step of incubating the single contiguous aqueous volume to generate the open linear DNA product may comprise:
i) digesting the first DNA molecule with the endonuclease by cleaving the cleavable target sequence (e.g. endonuclease target sequence) to generate the linear portion of the first DNA molecule;
ii) digesting the second DNA molecule with the endonuclease by cleaving the cleavable target sequence (e.g. endonuclease target sequence) to generate the linear portion of the second DNA molecule; and
iii) ligating the first adaptor molecule to a first end of the linear portion of the first DNA molecule, ligating the second end of the linear portion of the first DNA molecule to the first end of the linear portion of the second DNA molecule and ligating the second end of the linear portion of the second DNA molecule to the second adaptor molecule.

The invention provides a method for producing a partially closed linear DNA product, wherein the method comprises:
a) amplifying a first DNA template molecule (e.g. by rolling circle amplification) to generate a first DNA molecule, wherein the first DNA template molecule comprises at least one cleavable target sequence (e.g. endonuclease target sequence), and amplifying a second DNA template molecule (e.g. by rolling circle amplification) to generate a second DNA molecule, wherein the second DNA template molecule comprises at least one cleavable target sequence (e.g. endonuclease target sequence);
b) forming a single contiguous aqueous volume comprising the first DNA molecule, the second DNA molecule, an endonuclease, a ligase, and first and second adaptor molecules; and
c) incubating the single contiguous aqueous volume to generate the partially closed linear DNA product, wherein the partially closed linear DNA product comprises a linear double-stranded region, wherein the linear double-stranded region comprises a linear portion of the first DNA molecule and a linear portion of the second DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule (e.g. a DNA molecule) that comprises one or more nuclease-resistant nucleotides, and wherein the linear double-stranded region is closed at the second end by the second adaptor molecule (e.g. a DNA molecule).

The invention provides a method for producing a partially closed linear DNA product, wherein the method comprises:
a) amplifying a first DNA template molecule (e.g. by rolling circle amplification) to generate a first DNA molecule, wherein the first DNA template molecule comprises at least one cleavable target sequence (e.g. endonuclease target sequence) and wherein the first DNA molecule is a first concatemer, and amplifying a second DNA template molecule (e.g. by rolling circle amplification) to generate a second DNA molecule, wherein the second DNA template molecule comprises at least one cleavable target sequence (e.g. endonuclease target sequence) and wherein the second DNA molecule is a second concatemer;
b) forming a single contiguous aqueous volume comprising the first DNA molecule, the second DNA molecule, an endonuclease, a ligase, and first and second adaptor molecules; and
c) incubating the single contiguous aqueous volume to generate the partially closed linear DNA product, wherein the partially closed linear DNA product comprises a linear double-stranded region, wherein the linear double-stranded region comprises a linear portion of the first DNA molecule and a linear portion of the second DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule (e.g. a DNA molecule) that comprises one or more nuclease-resistant nucleotides, and wherein the linear double-stranded region is closed at the second end by the second adaptor molecule (e.g. a DNA molecule).

In the methods, the step of incubating the single contiguous aqueous volume to generate the partially closed linear DNA product may comprise:
i) digesting the first DNA molecule with the endonuclease by cleaving the cleavable target sequence (e.g. endonuclease target sequence) to generate the linear portion of the first DNA molecule;
ii) digesting the second DNA molecule with the endonuclease by cleaving the cleavable target sequence (e.g. endonuclease target sequence) to generate the linear portion of the second DNA molecule; and
iii) ligating the first adaptor molecule to a first end of the linear portion of the first DNA molecule, ligating the second end of the linear portion of the first DNA molecule to the first end of the linear portion of the second DNA molecule and ligating the second end of the linear portion of the second DNA molecule to the second adaptor molecule.

The linear double-stranded region may comprise a linear portion of the first DNA molecule ligated to a linear portion of the second DNA molecule. The linear portion of the first DNA molecule may be ligated directly to the linear portion of the second DNA molecule. The linear portion of the first DNA molecule may be ligated indirectly to the linear portion of the second DNA molecule. The linear portion of the first DNA molecule may be ligated to a linker sequence that is ligated to the linear portion of the second DNA molecule.

The first DNA template molecule may be amplified by rolling circle amplification to generate the first DNA molecule and/or the second DNA template molecule may be amplified by rolling circle amplification to generate the second DNA molecule.

In the methods, the first DNA molecule and/or the second DNA molecule generated in step (a) may not be purified prior to step (b). Preferably, the first DNA molecule and the second DNA molecule generated in step (a) are not purified prior to step (b).

In the methods, the DNA template molecules (e.g. the first DNA template molecule and the second DNA template molecule) are amplified either (i) separately, or (ii) together in a single contiguous aqueous volume.

The at least one cleavable target sequence may be a restriction endonuclease target sequence and the endonuclease may be a restriction endonuclease. The at least one cleavable target sequence may be a Type IIS restriction endonuclease target sequence and the endonuclease may be a Type IIS restriction endonuclease.

The endonuclease may be an RNA-guided DNA endonuclease.

Any of the methods provided herein may be a cell-free method.

The DNA product (e.g. the linear DNA product) may comprise a DNA cassette. The DNA cassette may be formed of a first cassette sequence and a second cassette sequence. The first DNA template molecule may comprise a first cassette sequence; and the second DNA template molecule may comprise a second cassette sequence. The linear double-stranded region may comprise the DNA cassette (i.e. the complete DNA cassette).

In the methods, the first DNA template molecule, the first DNA molecule and the linear portion of the first DNA molecule may each comprise a first cassette sequence; and the second DNA template molecule, the second DNA molecule and the linear portion of the second DNA molecule may each comprise a second cassette sequence. The linear double-stranded region may comprise the DNA cassette (i.e. the complete DNA cassette).

The skilled person would be aware of a range of options for the composition of the DNA cassette and sequence components that might be included in such a cassette. Various non-limiting options for the cassette are described herein.

Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The first cassette sequence may comprise a promoter. The promoter may be an inducible or constitutive promoter. The promoter may be a non-specific promoter. The promoter may be a CMV promoter, a CAG promoter, a Rous sarcoma virus (RSV) promoter, a simian virus 40 (SV40) promoter, a mammalian elongation factor 1α (EF1α) promoter, a MPSV (Moloney Murine Sarcoma Virus) promoter, a Human Ubiquitin C (UBC) promoter and/or a human phosphoglycerate kinase hPGK promoter. The promoter may be a tissue-specific promoter. The promoter may be a thyroxine binding globulin (TBG) promoter, a glial fibrillary acidic protein (GFAP) promoter and/or an actin promoter.

The second cassette sequence may comprise a coding sequence. The coding sequence may be at least 250, at least 500, at least 750, at least 1000, at least 1500, at least 2000, at least 3000, at least 4000, at least 5000, at least 7500, at least 10000, at least 15000 or at least 20000 base pairs (bp) in length. The coding sequence may be 250 to 20000, 500 to 15000, 750 to 10000, 1000 to 7500, 1500 to 5000, or 2000 to 4000 base pairs (bp) in length

The DNA cassette may comprise a promoter, a coding sequence and a 3' untranslated region e.g. a poly(A) tail. The DNA cassette may comprise a promoter, a coding sequence, a 3' untranslated region e.g. a poly(A) tail, and a translational termination sequence. The DNA cassette may comprise a promoter, a ribosome binding site, a coding sequence and a 3' untranslated region e.g. a poly(A) tail. The DNA cassette may comprise a promoter, a ribosome binding site, a coding sequence, a 3' untranslated region e.g. a poly(A) tail, and a translational termination sequence. The DNA cassette may comprise one or more of: a promoter; a ribosome binding site; a coding sequence; a 3' untranslated region e.g. a poly(A) tail; and a translational termination sequence.

The DNA cassette may further comprise an enhancer. The enhancer may be a CMV enhancer, an SV40 enhancer, a hTERT enhancer, an HIV enhancer, an LTR enhancer and/or a GATA enhancer.

The DNA cassette may comprise an insulator. The insulator may be a cis-regulatory element. The insulator may be at least 300, at least 400, at least 500, at least 1000, at least 1500 or at least 2000 nucleotides in length. The insulator may function either as an enhancer-blocker or a barrier, or both.

The DNA cassette may be an expression cassette. The DNA cassette may be a eukaryotic expression cassette e.g. a mammalian expression cassette or a plant expression cassette. The DNA cassette may be a prokaryotic expression cassette e.g. a bacterial expression cassette. The DNA cassette may be a viral expression cassette.

The DNA cassette may further comprise a reporter gene. The reporter gene may be an eGFP reporter gene, a luciferase reporter gene, or a β-glucuronidase reporter gene.

The DNA cassette may additionally comprise a sequence aiding protein expression, e.g. a cap-independent translation element such as an internal ribosome entry site (IRES).

The DNA cassette may comprise the sequence of a gene. The DNA cassette may comprise the sequence for at least two, three, four, or five genes.

The DNA cassette may comprise a sequence encoding an antigen. The DNA cassette may comprise sequences encoding at least 2, at least 3, at least 4, at least 5, at least 6, at least 7, at least 8, at least 9, at least 10, at least 15, at least 20, at least 30, at least 40, at least 50, at least 60, at least 70, at least 80, at least 90 or at least 100 antigens. The antigen(s) may be (a) self-antigen(s). The antigen(s) may be (a) neoantigen(s). The DNA cassette may comprise sequences encoding at least 5, at least 10, at least 20, at least 30, at least 40, at least 50, at least 60, at least 70, at least 80, at least 90, at least 100, at least 110 or at least 120 antigen (or neoantigen) units.

The DNA cassette may comprise (or encode) a repair template (or editing template). The repair template (or editing template) may be for use in CRISPR-Cas mediated homology directed repair (HDR). The DNA cassette may encode a CRISPR guide RNA.

The DNA cassette may further comprise a LoxP sequence, preferably two LoxP sequences. The two LoxP sequences may be oriented in the same direction. In this case, the DNA sequence between the two LoxP sequences may be excised as a circular loop of DNA. The two LoxP sequences may be oriented in opposite directions. In this case, the DNA sequence between the two LoxP sequences may be inverted. Preferably, the two LoxP sequences are in the same orientation (i.e. the same direction) in the cassette. One LoxP sequence may be at a first end of the cassette and the other LoxP sequence may be at a second end of the cassette. One LoxP sequence may be upstream and the other LoxP sequence may be downstream of the other sequence components of the cassette.

The DNA cassette may be a functional cassette i.e. it may have all sequence elements required for expression of a coding sequence.

As described herein, the DNA template molecules (each comprising a cassette sequence) may be amplified separately. The amplification of the second DNA template molecule may be performed in the presence of a methyltransferase or one or more methylated nucleotides. The amplification of the first DNA template molecule may be performed in the absence of a methyltransferase or one or more methylated nucleotides.

The first DNA molecule may comprise the unmethylated region and the second DNA molecule may comprise the methylated region.

The methods may comprise amplifying the first and second DNA template molecules to generate the first and second DNA molecules, wherein the DNA template molecules comprise at least one cleavable target sequence (e.g. endonuclease target sequence).

The first DNA template molecule may comprise a first cassette sequence and the second DNA template molecule may comprise a second cassette sequence.

The DNA template molecules may each comprise at least one cleavable target sequence. The cleavable target sequence may be an endonuclease target sequence. Thus, each DNA template molecule may comprise at least one endonuclease target sequence. Preferably, each DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a Bbsl, Bsal, BsmBI, BspQl, BtgZl, Esp3l, Sapl, Aarl, Acc36I, AcIWI, Acul, Ajul, Alol, AIw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3l, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI target sequence. The at least one cleavable sequence (e.g. endonuclease target sequence) may be a native cleavable sequence (i.e. a cleavable sequence present in the DNA template molecule(s)). Alternatively, the at least one cleavable sequence (e.g. endonuclease target sequence) may be introduced to the DNA template molecule prior to the amplification of the DNA template molecule.

The DNA template molecule used in the methods may be single-stranded or double-stranded. A single stranded DNA template molecule may be generated by denaturing a double stranded DNA template molecule. Thus, the methods may further comprise denaturing a double stranded DNA template molecule to generate a single stranded DNA template molecule. Denaturation may be achieved by any means known in the art including heat and/or chemical treatment (including organic solvents such as DMSO). Preferably, the DNA template molecule is double-stranded.

The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from a recombinase reaction, preferably a Cre recombinase reaction, or (ii) a circular DNA molecule obtained from a ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), the DNA template molecule may be circularized to produce a DNA template molecule suitable for use in the methods described herein.

Amplification (i.e. the step(s) of amplifying) may be performed: using a primer specific for the DNA template molecule; using a pair of primers specific for the DNA template molecule; or in the presence of a primase (such as *Thermus thermophilus (Tth)* PrimPol (TthPrimPol)). Primers specific for the DNA template molecule include single-stranded nucleic acids that are complementary to a target sequence in the DNA template molecule. A first primer of a set of primers may be complementary to the beginning of a target sequence in the DNA template molecule and a second primer of the set of primers may be complementary to the end of the target sequence in the DNA template molecule. In the case of a double-stranded DNA template, a first primer may be complementary to a first strand and a second primer may be complementary to a second strand.

Amplification (i.e. the step(s) of amplifying) may comprise in vitro or in vivo amplification. Preferably, the step(s) of amplifying is/are steps of in vitro amplification. For example, the step(s) of amplifying may be performed by rolling circle amplification (RCA), Multiple Annealing and Looping Based Amplification Cycles (MALBAC), polymerase chain reaction (PCR), nucleic acid sequence-based amplification (NASBA), loop-mediated isothermal amplification (LAMP), helicase-dependent amplification (HDA), multiple displacement amplification (MDA) or recombinase polymerase amplification (RPA). Preferably, amplification comprises isothermal amplification. More preferably, amplification comprises rolling circle amplification.

Rolling circle amplification may be performed without any primers (i.e. in the presence of a primase), or in the presence of a primer or multiple primers. For example, the primer may be a synthetic primer. The primers may be primers specific for a target sequence and/or random primers. Rolling circle amplification may be performed in the presence of a primase. The primase may be a primase (such as *Thermus thermophilus (Tth)* PrimPol (TthPrimPol)). Preferably, if the rolling circle amplification is performed without any primers, it is performed in the presence of a primase, such as TthPrimPol. If the rolling circle amplification is performed with a primase, it may also be performed in the presence of one or more primers (such as synthetic primers). Thus, rolling circle amplification may be performed with primers and with a primase. Amplification may be performed with a strand-displacing polymerase e.g. Phi29 DNA polymerase or a mutant thereof which retains functionality (e.g. QualiPhi^{®}, a chimeric form of Phi29 DNA polymerase from 4basebio). Amplification may be performed in the presence of nuclease-resistant nucleotide triphosphates, such as phosphorothioated nucleotide triphosphates. The incorporation of nuclease-resistant nucleotide triphosphates may render the amplified DNA product (e.g. DNA molecule) resistant to exonuclease digestion.

The first DNA molecule may comprise a first cassette sequence and the second DNA molecule may comprise a second cassette sequence.

The amplified DNA product(s) (e.g. the first and second DNA molecules) is/are preferably produced by rolling circle amplification.

The amplified DNA product(s) (e.g. the first and second DNA molecules) may be circular or branched.

The first DNA molecule may be a first concatemer and the second DNA molecule may be a second concatemer. The concatemers may be generated by rolling circle amplification. The first DNA molecule and the second DNA molecule may each comprise two or more copies of the same cassette sequence. The first DNA molecule and the second DNA molecule may each be a concatemer comprising multiple copies of same cassette sequence.

The first DNA molecule and the second DNA molecule may each comprise double-stranded regions and single-stranded regions. Preferably, the first DNA molecule and the second DNA molecule is/are double-stranded DNA molecules.

The first DNA molecule and the second DNA molecule may each comprise at least one cleavable sequence. Each cleavable sequence may be an endonuclease target sequence. Thus, the first DNA molecule and the second DNA molecule may each comprise at least one endonuclease target sequence. Preferably, first DNA molecule and the second DNA molecule may each comprise at least two endonuclease target sequences. The endonuclease target sequences may be the same or different. Preferably, the at least one endonuclease target sequence is a restriction endonuclease target sequence. Different restriction endonuclease target sequences are known to the skilled person. The cleavable target sequence may be a Type IIS restriction endonuclease target sequence. For example, the restriction endonuclease target sequence may be a Bbsl, Bsal, BsmBI, BspQl, BtgZl, Esp3l, Sapl, Aarl, Acc36I, AcIWI, Acul, Ajul, Alol, AIw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3l, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI target sequence. The at least one cleavable sequence (e.g. endonuclease target sequence) may be a native cleavable sequence (i.e. a cleavable sequence present in the DNA template molecule(s)). Alternatively, the at least one cleavable sequence (e.g. endonuclease target sequence) may be introduced into the DNA template molecule prior to the amplification of the DNA template molecule.

The first DNA molecule and/or the second DNA molecule may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the first DNA molecule and the second DNA molecule. The spacer may improve ligation efficiency of first and second adaptor molecules to the linear double-stranded region. The spacer may improve cell transfection yields.

The methods may comprise: forming a single contiguous aqueous volume comprising the first DNA molecule and the second DNA molecule, an endonuclease and a ligase; and incubating the single contiguous aqueous volume to generate the DNA product (e.g. the linear DNA product), wherein the DNA product (e.g. the linear DNA product) comprises a linear double-stranded region, wherein the linear double-stranded region comprises a linear portion of each of the amplified DNA products (e.g. a linear portion of the first DNA molecule and a linear portion of the second DNA molecule).

The linear portion of the first DNA molecule may comprise a first sequence of the DNA cassette and the linear portion of the second DNA molecule may comprise a second sequence of the DNA cassette.

The first DNA molecule may be digested to generate a linear portion of the first DNA molecule and the second DNA molecule may be digested to generate a linear portion of the second DNA molecule. The linear portion of the first DNA molecule and the linear portion of the second DNA molecule may each comprise a single cassette sequence. The term "single cassette sequence" as used herein is intended to encompass a product that does not comprise or consist of a plurality of cassette sequences. That is to say that the digested product (i.e. the linear portion) comprises only a single cassette sequence, which may for example comprise a single coding sequence of a gene of interest. The single cassette sequence may not comprise or consist of a plurality of tandem repeat sequences, and/or concatemeric DNA. The term "single cassette sequence" as used herein is intended to encompass a single copy of a sequence of the DNA cassette, for example, a single copy of the coding sequence or a single copy of a promoter sequence. Thus, the "single cassette sequence" may not encompass a cassette sequence that comprises or consists of multiple copies of the same DNA sequence linked in series.

The linear portions may have low dispersity (i.e. be substantially monodisperse). As used herein, the terms "low dispersity" and "monodisperse" are intended to encompass a collection of copies of digested DNA product of substantially the same size, i.e. the same length of the polynucleotide chain. That is to say, each linear portion may vary in size (i.e. number of bases in the polynucleotide chain) from the other linear portions from the same DNA molecule (e.g. the first DNA molecule or the second DNA molecule) by less than 10%, preferably by less than 5%.

The linear portion of the first DNA molecule and the linear portion of the second DNA molecule may each be at least 50, at least 100, at least 250, at least 500, at least 750, at least 1000, at least 2000, at least 3000, at least 4000, at least 5000, at least 6000, at least 7000, at least 8000, at least 9000, at least 10000, at least 11000, at least 12000, at least 13000, at least 14000, at least 15000, or at least 20000 base pairs long. Preferably, the linear portion of the first DNA molecule and the linear portion of the second DNA molecule are each at least 100 base pairs long. The linear portion of the first DNA molecule and the linear portion of the second DNA molecule may be 50 to 20000, 100 to 15000, 250 to 14000, 500 to 13000, 750 to 12000, 1000 to 11000, 2000 to 10000, 3000 to 9000, 4000 to 8000, or 5000 to 7000 base pairs long.

As used herein the term "linear double-stranded region" refers to a double stranded DNA comprising a linear portion of the first DNA molecule and a linear portion of the second DNA molecule.

The linear double-stranded region may comprise a DNA cassette (i.e. a complete DNA cassette).

The linear portion of the first DNA molecule may be ligated to the linear portion of the second DNA molecule to generate the linear double-stranded region.

The linear portion of the first DNA molecule and the linear portion of the second DNA molecule may each have an overhang. For example, linear portion of the first DNA molecule and the linear portion of the second DNA molecule may each comprise a 5' overhang at a first end and a 3' overhang at a second end. Each overhang may have at least 3 nucleotides (preferably from 4 to 6 nucleotides). The 5' overhang of a linear portion of the first DNA molecule may be complementary to the 3' overhang of a linear portion of the second DNA molecule (or vice versa). The 5' overhang of a linear portion of the first DNA molecule may anneal to the 3' overhang of a linear portion of the second DNA molecule (or vice versa).

Alternatively, the linear portion of the first DNA molecule and the linear portion of the second DNA molecule may each have blunt ends. The blunt ends may be ligated together and, in this way, the linear double-stranded region may be formed of a linear portion of the first DNA molecule and the linear portion of the second DNA molecule.

The first end and the second end of the linear double-stranded region may be resistant to nuclease digestion. Preferably, the first end and the second end of the linear double-stranded region are resistant to exonucleases that cleave the 3'-end nucleotides (e.g. exonuclease III) and/or exonucleases that cleave the 5'-end nucleotides (e.g. exonuclease VIII).

The linear double-stranded region may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 6 nucleotides). The overhang may be in the sense strand or the antisense strand of the linear double-stranded region.

The linear double-stranded region (or the linear portion of the first DNA molecule or the linear portion of the second DNA molecule) may comprise a plurality of nuclease-resistant nucleotides (e.g. phosphorothioated nucleotides) at internal positions in each strand. For example, the linear double-stranded region (or the linear portion of the first DNA molecule or the linear portion of the second DNA molecule) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 nuclease-resistant nucleotides (e.g. phosphorothioated nucleotides) at internal positions in each strand. Preferably, the linear double-stranded region (or the linear portion of the first DNA molecule or the linear portion of the second DNA molecule) comprises at least 2 nuclease-resistant nucleotides (e.g. phosphorothioated nucleotides) at internal positions in each strand. The internal positions may not be located between the second and penultimate nucleotide of the linear double-stranded region (or DNA cassette).

The first end of the linear double-stranded region may be complementary to a portion of the first adaptor molecule. The second end of the linear double-stranded region may be complementary to a portion of the second adaptor molecule. The first end and/or the second end of the linear double-stranded region may be generated by endonuclease digestion.

The DNA product (e.g. the linear DNA product) produced by the methods described herein has enhanced resistance to exonuclease digestion (e.g. exonuclease III digestion). The enhanced resistance to exonuclease digestion may extend the life of the DNA product (e.g. the linear DNA product) in a cell (i.e. the DNA product (e.g. the linear DNA product) has enhanced resistance to intracellular exonucleases) and in a cell-free system (i.e. the DNA product (e.g. the linear DNA product) has enhanced resistance to extracellular exonucleases). The enhanced resistance to exonuclease digestion may be provided by appending (e.g. ligating) a first adaptor molecule to a first end of the linear double-stranded region and a second adaptor to a second end of the linear double stranded region. In this regard, the entire content of WO2023/006978 A1 is incorporated herein by reference.

As described herein, a first adaptor molecule and a second adaptor molecule may be included in the single contiguous aqueous volume. The first adaptor molecule may be ligated to the first end of the linear double-stranded region and the second adaptor molecule may be ligated to the second end of the linear double-stranded region.

The first adaptor molecule may comprise a sequence of the DNA cassette (i.e. a cassette sequence). For example, the first adaptor molecule may comprise a promoter or a portion thereof. The second adaptor molecule may comprise a sequence of the DNA cassette (i.e. a cassette sequence). The second adaptor molecule may comprise a poly(A) tail, or a portion thereof.

The first adaptor molecule and/or the second adaptor molecule may comprise a GC content of at least 50%, at least 60%, at least 65%, at least 70%, at least 75%, at least 80%, at least 85%, at least 90%, at least 95%, at least 100%. Preferably, the first adaptor molecule and/or the second adaptor molecule may have a GC content of at least 60%. More preferably, the first adaptor molecule and/or the second adaptor molecule may have a GC content of at least 65%. GC content may refer to the percentage of bases that are either guanine or cytosine.

The first adaptor molecule or the second adaptor molecule may comprise a sequence of a tandem repeat (with the repeat sequence of the tandem repeat being provided by an adjacent cassette sequence of the DNA cassette).

The first adaptor molecule and/or the second adaptor molecule may comprise an enhancer or a terminator.

The first adaptor molecule and/or the second adaptor molecule may comprise a long terminal repeat (LTR) or a repeat sequence thereof.

In the methods, the first adaptor molecule and the second adaptor molecule may both be linear adaptor molecules comprising nuclease resistant nucleotides, and an open linear DNA product may be produced.

In the methods, the first adaptor molecule and the second adaptor molecule may each comprise a hairpin or a stem-loop, and a closed linear DNA product may be produced.

In the methods, the first adaptor molecule may be a linear adaptor molecule comprising nuclease resistant nucleotides and the second adaptor molecule may comprise a hairpin or a stem-loop (or vice versa), and a partially closed linear DNA product may be produced. The partially closed linear DNA product may be a partially covalently closed linear DNA product.

The adaptor molecules may provide protection from digestion by exonucleases that cleave the 3'-end nucleotides (e.g. exonuclease III) and exonucleases that cleave the 5'-end nucleotides (e.g. exonuclease VIII).

The first and second adaptor molecules may be identical molecules, or they may be different molecules. For example, the first adaptor molecule and/or the second adaptor molecule may comprise a hairpin. The first adaptor molecule and/or the second adaptor molecule may be double-stranded linear nucleic acid molecules comprising one or more nuclease-resistant nucleotides. The first adaptor molecule may comprise a hairpin and the second adaptor molecule may be a double-stranded linear nucleic acid molecule comprising one or more nuclease-resistant nucleotides. The linear DNA product produced by the methods described herein may be resistant to nuclease (e.g. exonuclease) digestion.

The first adaptor molecule may be a nucleic acid adaptor molecule (e.g. DNA). The second adaptor molecule may be a nucleic acid adaptor molecule (e.g. DNA). The first adaptor molecule and/or the second adaptor molecule may be a synthetic adaptor molecule. The first adaptor molecule and/or the second adaptor molecule may not be a plasmid or a vector DNA.

The first adaptor molecule and/or the second adaptor molecule may comprise an overhang. For example, the first adaptor molecule and/or the second adaptor molecule may comprise a 5' overhang or a 3' overhang. The first adaptor molecule and/or the second adaptor molecule may comprise a blunt end. The overhang may have at least 3 nucleotides (preferably from 4 to 6 nucleotides). The overhang of the first adaptor molecule and/or the second adaptor molecule may be complementary to the first and/or second end of the linear double-stranded region. The overhang of the first adaptor molecule and/or the second adaptor molecule may anneal to the first and/or second end of the linear double-stranded region.

The first adaptor molecule and/or the second adaptor molecule may comprise a self-complementary element which creates a loop, such as a hairpin loop or a stem-loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4 to 6 nucleotides. Each end of the linear double-stranded region may comprise a 3' or a 5' overhang.

The first adaptor molecule and/or the second adaptor molecule may comprise a single-stranded portion. The single-stranded portion may form a hairpin or a stem-loop. Thus, the first adaptor molecule and/or the second adaptor molecule may comprise a loop portion. The single-stranded portion may comprise at least 2, at least 3, at least 4, at least 5, at least 6, at least 7, at least 8, at least 9, at least 10, at least 15, at least 20, at least 25, at least 30, at least 35, at least 40, at least 45 or at least 50 nucleotides. The single-stranded portion may comprise 2-50, 3-45, 4-40, 5-35, 6-30, 7-25, 8-20 or 9-15 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the linear double-stranded region (which may comprise a 3'-OH group at first and/or second ends). The first adaptor molecule and/or the second adaptor molecule may comprise a 3'-OH. The 3'-OH may facilitate ligation to the linear double-stranded region (which may comprise a 5' phosphate at first and/or second ends).

The first adaptor molecule may comprise a portion (e.g. the overhang) that is complementary to the first end of the linear double-stranded region. The second adaptor molecule may comprise a portion (e.g. the overhang) that is complementary to the second end of the linear double-stranded region. The first adaptor molecule may comprise a portion that anneals to the first end of the linear double-stranded region. The second adaptor molecule may comprise a portion that anneals to the second end of the linear double-stranded region. The first adaptor molecule may comprise a portion that is complementary and anneals to the first end of the linear double-stranded region. The second adaptor molecule may comprise a portion that is complementary and anneals to the second end of the linear double-stranded region.

The portion that is complementary or anneals to the first or second end of the linear double-stranded region may be a 5' overhang or a 3' overhang of the first and/or second adaptor molecule. The overhang of the first adaptor molecule may be complementary to the first end of the linear double-stranded region and/or the overhang of the second adaptor molecule may be complementary to the second end of the linear double-stranded region. The overhang of the first adaptor molecule may anneal to the first end of the linear double-stranded region and/or the overhang of the second adaptor molecule may anneal to the second end of the linear double-stranded region. The overhang of the first adaptor molecule may be complementary to and anneal to the first end of the linear double-stranded region and/or the overhang of the second adaptor molecule may be complementary to and anneal to the second end of the linear double-stranded region.

The first adaptor molecule and/or the second adaptor molecule may not comprise a Type IIS endonuclease target sequence. The first adaptor molecule and/or the second adaptor molecule may not comprise Bbsl, Bsal, BsmBI, BspQl, BtgZl, Esp3I,SapI, Aarl, Acc36I, AcIWI, Acul, Ajul, Alol, AIw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, Semi, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI target sequences.

The first adaptor molecule and/or the second adaptor molecule may comprise one or more locked nucleic acids (LNAs).

The first adaptor molecule and/or the second adaptor molecule may comprise one or more nuclease-resistant nucleotides (i.e. protected nucleotides), such as phosphorothioated nucleotides. The nuclease-resistant nucleotides may be located in the single-stranded portion (e.g. hairpin portion) or the double-stranded portion of the adaptor molecules. The nuclease-resistant nucleotides may be located in the overhang portion of the adaptor molecules.

The first adaptor molecule and/or the second adaptor molecule may confer resistance to 3'-end exonuclease digestion (e.g. by exonuclease III) and/or 5'-end exonuclease digestion (e.g. by exonuclease VIII).

The closing of the linear double-stranded region at the first end may generate a first closed end of the closed linear DNA product. The closing of the linear double-stranded region at the second end may generate a second closed end of the closed linear DNA product. The first closed end and the second closed end of the closed linear DNA product may be resistant to nuclease digestion. The nuclease digestion may be exonuclease digestion. Preferably, the first closed end and the second closed end of the closed linear DNA product confer resistance to 3'-end exonuclease digestion (e.g. by exonuclease III) and/or 5'-end exonuclease digestion (e.g. by exonuclease VIII).

The first and second adaptor molecules may comprise one or more nuclease-resistant nucleotides (e.g. phosphorothioated nucleotides), such that, once the adaptor molecules are appended (e.g. ligated) to the linear double-stranded region, the linear DNA product is resistant to nuclease digestion or has improved or enhanced resistance to nuclease digestion. The linear DNA product may be resistant to 3'-end exonuclease digestion (e.g. by exonuclease III) and/or 5'-end exonuclease digestion (e.g. by exonuclease VIII).

Each adaptor molecule may comprise a plurality of phosphorothioated nucleotides. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

Each adaptor molecule may be double-stranded (e.g. double-stranded DNA). Each adaptor molecule may comprise a portion that is double-stranded (e.g. double-stranded DNA).

The first and/or second adaptor molecules may each comprise at least 3, at least 4, at least 5, at least 6, at least 7, at least 8, at least 9, at least 10, at least 11, at least 12, at least 13, at least 14, at least 15, or at least 16 base pairs.

Each adaptor molecule may comprise a plurality of phosphorothioated nucleotides in each strand. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

Each adaptor molecule may comprise a plurality of phosphorothioated nucleotides at internal positions in each strand. For example, each adaptor molecule may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, each adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

Each adaptor molecule may comprise at least 5%, at least 10%, at least 20%, at least 30%, at least 40%, at least 50%, at least 60%, at least 70%, at least 80%, at least 90%, at least 95%, or at least 100% of nuclease-resistant nucleotides.

The nuclease-resistant nucleotides (i.e. nucleotides resistant to exonuclease digestion) may be phosphorothioated nucleotides of at least one type. For example, the at least one type of phosphorothioated nucleotides is α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The adaptor molecules may comprise at least four types of phosphorothioated nucleotides. For example, the at least four types of nuclease-resistant nucleotides are α-S-dATP, α-S-dCTP, α-S-dGTP and α-S-dTTP.

Although the adaptor molecules are described herein as comprising phosphorothioated nucleotides, the skilled person would appreciate that the adaptor molecules may instead comprise any molecules that provide resistance to nuclease digestion (e.g. exonuclease digestion). For example, the adaptor molecules may comprise nuclease-resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence.

The functional portion may be a binding molecule. The term "binding molecule" refers to any molecule capable of binding to the DNA product (e.g. the linear DNA product) described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product (e.g. the linear DNA product) by binding to capture molecules (e.g. capture antibodies bound by protein-protein interactions). The functional portion may bind to a cell target, for example, a cell receptor.

The functional portion may be a label. The 'label' can be any chemical entity which enables the detection of the double-stranded nucleic acid molecule via physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product (e.g. the linear DNA product) to a specific location in a cell. The targeting sequence may be used to increase transfection efficiency of non-viral gene delivery by virtue of enhanced nuclear import of the DNA product (e.g. the linear DNA product). For example, the targeting sequence may be a DNA nuclear targeting sequence (i.e. a recognition sequence for endogenous DNA-binding proteins), such as a SV40 enhancer sequence (preferably downstream from the DNA cassette).

To facilitate detection and/or quantification of the DNA product (e.g. the linear DNA product), the functional portion may comprise a fluorophore, a radioactive compound or a barcode.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an inverted terminal repeat (ITR) sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different (viral) serotypes. An inverted terminal repeat sequence may comprise a terminal resolution site and a Rep binding site.

The first adaptor molecule and/or the second adaptor molecule may comprise an aptamer.

The endonuclease may be a restriction endonuclease (such as a Type II restriction endonuclease), RNA-guided DNA endonuclease, meganuclease or homing endonuclease. The Type II restriction endonuclease may be a Type IIP restriction endonuclease (e.g. EcoRl, Hindlll, BamHI or Notl) or a Type IIS restriction endonuclease (e.g. Bsal, Bbvl, Fokl or Sapl). The RNA-guided DNA endonuclease may be an endonuclease of Class 2 e.g. Class 2 Type V. The RNA-guided DNA endonuclease may be Cas12a. The RNA-guided DNA endonuclease may be Cpf1 or Mad7. Preferably, the RNA-guided DNA endonuclease is Cpf1. The homing endonuclease may be I-Ceul, I-Scel, Pl-Pspl or Pl-Scel.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQl, BtgZl, Esp3I, Sapl, Aarl, Acc36I, AcIWI, Acul, Ajul, Alol, AIw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI restriction enzyme.

Type IIS restriction endonucleases cleave the amplified DNA product outside of the recognition sequence (i.e. an endonuclease target sequence), which means that the recognition sequence (i.e. endonuclease target sequence) is not included in the DNA product (e.g. the linear DNA product).

The endonuclease may be at least 2, at least 3, at least 4 or at least 5 different endonucleases.

The ligase may be a DNA ligase, such as a T4 DNA ligase, T7 DNA ligase, mammalian DNA ligase I, III and IV; Taq DNA ligase, Tth DNA ligase, or E. *coli* DNA ligase.

The step of incubating the single contiguous aqueous volume to generate the DNA product (e.g. the linear DNA product) may comprise: digesting the first DNA molecule and the second DNA molecule with an endonuclease by cleaving the cleavable target sequence (e.g. endonuclease target sequence) to generate a linear portion of the first DNA molecule and a linear portion of the second DNA molecule; and ligating the linear portion of the first DNA molecule to the linear portion of the second DNA molecule to generate the linear double-stranded region.

The step of incubating the single contiguous aqueous volume may be performed under conditions that promote appending (or linking) of the first and second adaptor molecules to the linear double-stranded region to produce the DNA product (e.g. the linear DNA product). The appending may be performed by creating a covalent link between the first and/or second adaptor molecule and the first and/or second end of the linear double-stranded region.

The first adaptor molecule may be appended to a first end of the linear double-stranded region and the second adaptor molecule may be appended to a second end of the linear double-stranded region. The appending (or linking) of the of the first adaptor molecule and/or second adaptor molecule may be performed by hybridization and/or ligation of the first adaptor molecule and/or second adaptor molecule to the end(s) of the linear double-stranded region. The first adaptor molecule may be hybridized and/or ligated to the first end of the linear double-stranded region. The second adaptor molecule may be hybridized and/or ligated to the second end of the linear double-stranded region. The appending of the first adaptor molecule and/or the second adaptor molecule may be performed by both hybridization and ligation of the adaptor molecules to the ends of the linear double-stranded region. The first adaptor molecule may be hybridized and ligated to the first end of the linear double-stranded region. The second adaptor molecule may be hybridized and ligated to the second end of the linear double-stranded region. The hybridization is based on complementarity of a portion of the first and/or second adaptor molecules to the first and/or second end of the linear double-stranded region. The appending may be performed via a linker or spacer molecule which facilitates joining of the adaptor molecule to the first and/or second end of the linear double-stranded region.

The linker or spacer may be at least 10, at least 20, at least 30, at least 40, at least 50, at least 60, at least 70, at least 80, at least 90, at least 100, at least 125, at least 150, at least 175, or at least 200 base pairs long.

The step of incubating the single contiguous aqueous volume may be performed under conditions that promote digestion of the amplified DNA products to produce the linear portions of the amplified DNA products. The digestion of the amplified DNA products to produce the linear portions of the amplified DNA products may be performed at a first temperature of 1°C-100°C, 1°C-80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the linear portions of the first and second DNA molecules (and optionally the first and second adaptor molecules). The ligation may be at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% efficient. For example, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% of the linear portions of the first and second DNA molecules may be incorporated into DNA products (e.g. the linear DNA products). Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining unprotected DNA molecules and adaptor molecules excess.

For example, the first and second DNA molecules generated by rolling circle amplification are first quantified so that the amount of the amplified products used as the starting material during the digestion/ligation reaction is known. After all the enzymatic reactions, the DNA product (e.g. the linear DNA product) is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the linear portions of the first and second DNA molecules (and, optionally, the first and second adaptor molecules) may be performed at a second temperature of 1°C -90°C, 2°C - 70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C and the second temperature is 14°C-18°C. Using these conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or E. *coli* DNA ligase.

The step of incubating the single contiguous aqueous volume may be performed isothermally. The step of incubating the single contiguous aqueous volume may comprise incubating at a constant temperature. The constant temperature promotes simultaneous digestion of the amplified DNA products (e.g. DNA molecules) to produce the linear portions of amplified DNA products (e.g. DNA molecules) and ligation of the linear portions of the amplified DNA products (e.g. DNA molecules) and the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous volume does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C-33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The method may further comprise (after the steps of amplification and before the step of forming a single contiguous aqueous volume) a step of heat-deactivation. The step of heat-deactivation may be performed under conditions sufficient to inactivate the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

The inventors of the present application have surprisingly discovered that large concatemeric products of rolling circle amplification reactions can be used to produce DNA products (e.g. linear DNA products). This is surprising as the product of the rolling circle amplification has high viscosity and typically has to undergo purification steps before it can be utilized for downstream applications.

In the method described herein, after the step of amplifying (step (a)) the step of forming a single contiguous aqueous volume (step (b)) may be performed without purifying the amplified DNA products (i.e. the first DNA molecule and the second DNA molecule). That is to say that the step of forming a single contiguous aqueous volume (step (b)) may be performed directly after the step of amplifying (step (a)).

Optionally, the first amplification product (i.e. the first DNA molecule) and/or the second amplification product (i.e. the second DNA molecule) may be heat-deactivated. The step of forming a single contiguous aqueous volume (step (b)) may be performed directly after the step of heat-deactivation.

In this regard, the inventors of the present application have surprisingly discovered that large concatemeric products of rolling circle amplification reactions can be used to produce DNA products (e.g. the linear DNA products) without the need for a purification step. This is surprising as the product of the rolling circle amplification has high viscosity and typically has to undergo purification steps before it can be utilized for downstream applications.

The inventors of the present application have discovered a method which requires very few steps to produce the DNA product (e.g. the linear DNA product) described herein. The methods described herein are very time efficient. This is, in part, due to the fact that a step of purification is not required after the step(s) of amplifying.

The method described herein in which the step of forming a single contiguous aqueous volume (step (b)) is performed without purifying the amplified DNA products (i.e. the first DNA molecule and the second DNA molecule) may produce a higher yield of the DNA product (e.g. the linear DNA product) when compared to a method in which the amplified DNA products are purified before the step of forming a single contiguous aqueous volume (step (b)).

The method may further comprise, after the step of incubating the single contiguous aqueous volume (step (c)), a step of purifying the DNA product (e.g. the linear DNA product).

The method may further comprise, after the step of incubating the single contiguous aqueous volume (step (c)), a step of nuclease digestion. The nuclease digestion may be exonuclease digestion, such as exonuclease I and/or exonuclease III digestion. The step of nuclease digestion may take place before or after the step of purifying the DNA product (e.g. the linear DNA product). The step of nuclease digestion may allow for removal of any double-stranded DNA molecules and/or adaptor molecules that have not been used to produce DNA products (e.g. the linear DNA products). The method may comprise incubating the single contiguous aqueous volume with a nuclease (e.g. an exonuclease).

The method may comprise: (d) incubating the single contiguous aqueous volume with a nuclease (e.g. an exonuclease); and (e) purifying the DNA product (e.g. the linear DNA product). The method may comprise: (d) purifying the DNA product (e.g. the linear DNA product); and (e) incubating the product of step (d) with a nuclease (e.g. an exonuclease).

The step of incubating with a nuclease may be performed at a temperature of 5-90°C, 10-80°C, 15-70°C, 20-60°C, 25-50°C, 30-45°C or 35-40°C. The step of incubating with a nuclease may be performed for at least 10, at least 20, at least 30, at least 40, at least 50, or at least 60 min. The step of incubating with a nuclease may be performed at two different temperatures. For example, the step of incubating with a nuclease may be performed at 15-40°C for 10-60 minutes followed by a temperature of 60-90°C for 10-30 min. The higher temperature typically inactivates the nuclease (e.g. exonuclease). Thus, the method may further comprise a step of inactivating the nuclease (e.g. exonuclease). The step of incubating with a nuclease may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The method may further comprise contacting the single contiguous aqueous volume (or the product of any of the above steps) with Proteinase K.

As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to a DNA sequence.

As used herein the term "nuclease-resistant nucleotide" or "protected nucleotide" is intended to encompass any type of nucleotide that provides or enhances resistance to nuclease digestion (especially exonuclease digestion).

The nuclease-resistant nucleotides (i.e. nucleotides resistant to exonuclease digestion) may be phosphorothioated nucleotides. As used herein, the term "phosphorothioated nucleotide" refers to a nucleotide that has an altered phosphate backbone, wherein the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nuclease-resistant nucleotides (i.e. nucleotides resistant to exonuclease digestion) may be 2'-O-methyl nucleotides or 2'-O-methoxyethyl (MOE) nucleotides. The nuclease-resistant nucleotides may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

### DNA products

The invention also provides a deoxyribonucleic acid (DNA) product comprising an unmethylated region and a methylated region. Preferably, the DNA product is linear.

The invention also provides a deoxyribonucleic acid (DNA) product comprising an unmethylated region and a methylated region, wherein the unmethylated region comprises a promoter sequence, and wherein the methylated region comprises one or more methylated CpG, CpA, CpT and/or CpC sites.

The unmethylated region may be 5' to the methylated region in the DNA product. The unmethylated region may comprise CpG sites. The unmethylated region may comprise complementary sense and antisense strands. The methylated region may comprise a coding sequence encoding a gene of interest. The methylated region may be methylated on substantially all CpG, CpA, CpT and/or CpC sites. The methylated region may comprise complementary sense and antisense strands.

The invention also provides a deoxyribonucleic acid (DNA) product comprising an unmethylated region and a methylated region, wherein the unmethylated region comprises a promoter sequence, and wherein the methylated region comprises one or more methylated CpG sites.

The unmethylated region may be 5' to the methylated region in the DNA product. The unmethylated region may comprise CpG sites. The unmethylated region may comprise complementary sense and antisense strands. The methylated region may comprise a coding sequence encoding a gene of interest. The methylated region may be methylated on substantially all CpG sites. The methylated region may comprise complementary sense and antisense strands.

The invention also provides a deoxyribonucleic acid (DNA) product comprising an unmethylated region and a methylated region, wherein the unmethylated region comprises a promoter sequence, and wherein the methylated region comprises one or more methylated CpA sites.

The unmethylated region may be 5' to the methylated region in the DNA product. The unmethylated region may comprise CpA sites. The unmethylated region may comprise complementary sense and antisense strands. The methylated region may comprise a coding sequence encoding a gene of interest. The methylated region may be methylated on substantially all CpA sites. The methylated region may comprise complementary sense and antisense strands.

The DNA product may be linear and comprise a closed loop and/or one or more nuclease-resistant nucleotides at one or both ends.

### Kits

The invention provides a kit for performing the methods described herein.

The invention provides a kit comprising:
a) means for generating a second DNA molecule comprising a methylated region from a second DNA template molecule or precursor DNA molecule; and
b) an enzyme for appending a first DNA molecule comprising an unmethylated region to the second DNA molecule.

Means for generating a second DNA molecule comprising a methylated region from a second DNA template molecule or precursor DNA molecule may comprise a polymerase and methylated nucleotide triphosphates; or a methyltransferase.

An enzyme for appending a first DNA molecule comprising an unmethylated region to the second DNA molecule may comprise a ligase, recombinase, topoisomerase or polymerase.

The invention provides a kit comprising:
(A)
   (a) a polymerase
   (b) methylated nucleotide triphosphates; and
   (c) a ligase; or
(B)
   (a) a methyltransferase; and
   (b) a ligase.

The kit may further comprise an endonuclease. The kit may further comprise a first DNA template molecule comprising an unmethylated region comprising a promoter sequence. The kit may further comprise a methyl donor such as S-Adenosyl methionine. The kit may further comprise at least one buffer. The kit may further comprise first and second adaptor molecules; an endonuclease; and a ligase.

### Methods for transcription and protein expression

The invention also provides a method for *in vitro* transcription of a DNA product, the method comprising:
(a) providing a DNA product, wherein the DNA product is the DNA product described herein, or wherein the DNA product is obtainable or produced by the methods described herein;
(b) contacting the DNA product with a polymerase; and
(c) producing a transcription product encoded by the DNA product.

The invention provides a method for in vitro transcription of a DNA product, wherein the method comprises contacting the DNA product produced or obtainable by the methods described herein with a (RNA) polymerase and producing a transcription product encoded by the protected DNA product.

The invention provides a method for producing a protein, wherein the method comprises introducing the DNA product, produced or obtainable by the methods described herein, into a cell (e.g. a prokaryotic cell or a eukaryotic cell) or a cell-free expression system to generate a protein encoded by the protected DNA product.

The invention provides a method for producing a protein, wherein the method comprises:
(a) producing a DNA product by any of the methods described herein; and
(b) introducing the DNA product into a cell (e.g. a prokaryotic cell or a eukaryotic cell) or a cell-free expression system to generate a protein encoded by the DNA product.

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The protected DNA product may comprise a cassette. The desired protein might be encoded by the cassette. The step of introducing the protected DNA product into a cell may be performed in vivo or in vitro. The nuclease-resistant nucleotides (i.e. protected nucleotides) may be any protected nucleotides described herein.

### Methods for cell transfection and cell transfection compositions

The invention provides a method for cell transfection of a DNA product produced or obtainable by any of the methods described herein, into a cell.

The invention provides a method for cell transfection of a DNA product into a cell, wherein the method comprises:
(a) producing a DNA product by any of the methods described herein;
(b) contacting a cell with the DNA product; and
(c) transfecting the DNA product into the cytosol of the cell.

The invention provides a cell transfection composition comprising a DNA product produced by (or obtainable by) the methods described herein.

The cell transfection composition may or may not comprise a carrier e.g. an agent or a formulation. Preferably, the carrier promotes accumulation of the DNA product at a target site and/or protects the DNA product from undesirable interactions with biological milieu components and/or protects the DNA product from metabolism and/or degradation.

The invention further provides a cell transfection method comprising contacting (in vitro) a cell to be transfected with a DNA product produced or obtainable by the methods of the invention, and wherein the DNA product is transfected into the cytosol of the cell.

The cell or cells to be transfected may be provided in a cell culture medium (e.g. in a Petri dish, culture vessel or well, etc). The DNA product may be added directly to the cell culture medium or the cells may be added to a solution, such as saline, a buffered solution or a cell culture medium, comprising the DNA product.

The carrier may be a viral carrier or a non-viral carrier. Viral carriers include a lentiviral vector, an adenoviral vector, a retroviral vector, or an adeno associate viral vector for delivery of the DNA product. Non-viral carriers (or vectors) include complexing the DNA product with a cationic agent such as a cationic cell penetrating peptide (CPP); a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the DNA product. The carrier may be a nanoparticulate formulation used to encapsulate the DNA product.

The carrier may be a modification of the DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the DNA product at a target site and/or protects the DNA product from metabolism and/or degradation.

The invention further provides a cell obtainable by the methods of the invention. Thus, the cell may comprise a DNA product produced or obtainable by the methods of the invention.

The invention further provides a cell transfected with a DNA product produced or obtainable by the methods of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a DNA product may be performed in vivo. For example, the DNA product may be administered to an organism (e.g. a subject) in need thereof. The organism (e.g. the subject) may be an animal, such as mammal (e.g. a human), a fungus, a micro-organism, or a plant.

Any or all of the DNA products described herein may be delivered to a cell via delivery particles such as liposomes, nanoparticles, exosomes, macrovesicles, viral or non-viral vectors. Any or all of the DNA products described herein may be delivered to a cell using a gene-gun. Any or all of the DNA products described herein may be delivered to a cell by electroporation. Any or all of the DNA products described herein may be delivered to a cell by hydrodynamic needle. The DNA products described herein may be delivered to a cell without a carrier.

The nanoparticle is preferably a self-assembled nanoparticle. The nanoparticle may be a nanoparticle which is produced by a process in which pre-existing components (e.g. a lipid component, a DNA product described herein) form an organized structure as a consequence of specific, local interactions among the components themselves, without external direction.

The DNA product and the lipid component may reversibly interact to form a self-assembled nanoparticle. The DNA product and the lipid component may reversibly interact in the self-assembled nanoparticle through intermolecular forces. The DNA product and the lipid component may reversibly interact in the self-assembled nanoparticle through non-covalent interactions. The DNA product and the lipid component may reversibly interact in the self-assembled nanoparticle through hydrogen bonds, van der Waals, hydrophobic, and/or electrostatic interactions. The DNA product and the lipid component may not be conjugated or linked by forces other than inter-molecular forces in the self-assembled nanoparticle.

### Pharmaceutical compositions and methods for producing pharmaceutical compositions

The invention also provides a pharmaceutical composition comprising a DNA product as described herein and a pharmaceutically acceptable carrier or excipient. The product in the pharmaceutical composition may be obtainable or produced by the methods of the invention.

The invention also provides a DNA product as described herein for use in therapy. The product may be obtainable or produced by the methods of the invention.

The invention also provides a vaccine comprising a DNA product as described herein. The product may be obtainable or produced by the methods of the invention.

The invention provides a pharmaceutical composition comprising a DNA product described herein, and a pharmaceutically acceptable carrier or excipient.

The invention provides a pharmaceutical composition comprising a DNA product produced or obtainable by the methods described herein, and a pharmaceutically acceptable carrier or excipient.

The invention provides a method for producing a pharmaceutical composition comprising the DNA product, wherein the method comprises performing the methods described herein to produce the DNA product and formulating the resulting DNA product with a pharmaceutically acceptable carrier or excipient.

Thus, the invention provides a method for producing a pharmaceutical composition, wherein the method comprises:
(a) producing a DNA product by any of the methods described herein;
(b) formulating the DNA product with a pharmaceutically acceptable carrier or excipient.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the DNA product can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes).

Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### Uses and Applications

The invention provides a use of a DNA product as described herein in the production of viral or non-viral delivery system.

The invention provides a use of a DNA product in the production of viral or non-viral delivery system, wherein the DNA product is produced or obtainable by performing the methods described herein.

The invention provides a viral or non-viral delivery system comprising a DNA product as described herein. The invention provides a viral or non-viral delivery system comprising a DNA product, wherein the DNA product is produced or obtainable by performing the method described herein.

### Viral vectors

Methods of producing viral vectors, such as AAV vectors, are known in the art. The most widely used method involves the co-transfection of HEK293 by three bacterial plasmids. The first plasmid encodes the Rep and *Cap* element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli;* 3) the incorporation of the plasmid backbone into the viral capsid (e.g. AAV capsid) might be challenging (e.g. issues with antibiotic resistance markers). Methods of producing viral vectors may be using other cell lines. For example, a cell line suitable for production of viral vectors may be a Vero cell, or any other stable cell line. Together these represent the main bottleneck in viral vector production (e.g. AAV production). Thus, the methods described herein provide a protected DNA product suitable for use in production of viral vectors. The DNA product overcomes the above issues with plasmid vectors.

The invention provides a method of producing a viral vector, the method comprising introducing the DNA product produced or obtainable by the methods described herein into a cell under conditions such that the viral vector is produced. The DNA product may encode at least one element required for the production of the viral vector. For example, the DNA product may encode Rep and/or Cap elements. The DNA product may encode the helper plasmid elements. The DNA product may encode Rep, Cap and helper plasmid elements. The DNA product may encode a transgene. The DNA product may encode Rep, Cap, helper plasmid elements and/or a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. the DNA product) to a cell, comprising contacting the viral vector produced by the methods described herein with the cell. The cell may be an animal cell, preferably mammal cell, such as human cell.

The invention also provides a cell obtainable by the methods described herein.

### Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the DNA product produced or obtainable by the methods described herein is suitable for use in production of non-viral vectors. The DNA product produced or obtainable by the methods of the invention overcomes the issues of using plasmid vectors in non-viral vector preparation. For example, the DNA product, unlike plasmid DNA, does not comprise a bacterial backbone, allowing more transgene copies per mg of DNA. In addition, the DNA product does not comprise antibiotic resistant genes and bacterial contaminants. Moreover, the DNA product provides a prolonged transgene expression (due to the unmethylated region and the methylated region), and a more cost-effective process of non-viral vector production.

The invention provides a method of delivering the non-viral vector to a cell, comprising contacting the non-viral vector comprising the DNA product with the cell. The cell may be an animal cell, preferably mammal cell, such as human cell.

The invention also provides a cell obtainable by the methods described herein.

### General therapeutic and diagnostic uses

The DNA product produced by the methods described herein is particularly suitable for use in therapy. The invention provides a DNA product as described herein for use in therapy. The invention provides a DNA product obtainable by the method described herein for use in therapy. The DNA product may encode a sequence of a therapeutic protein, a part of a vaccine, or an element of a genetic engineering mechanism, which is used to treat a disease or infection in a subject.

The invention further provides the DNA product as described herein for use as a medicament. The invention further provides the DNA product produced by the methods described herein for use as a medicament. The invention further provides the DNA product obtainable by the methods described herein for use as a medicament. The invention also provides the use of a DNA product as described herein for the manufacture of a medicament for treating a disease. The invention also provides the use of a DNA product produced by the methods described herein for the manufacture of a medicament for treating a disease. The invention also provides the use of a DNA product obtainable by the methods described herein for the manufacture of a medicament for treating a disease.

The DNA product may encode a sequence of a therapeutic protein, a part of a vaccine, or an element of a genetic engineering mechanism, which is used to treat a disease or infection in a subject.

The invention further provides the DNA product produced by the methods described herein for use in treating a disease.

The invention also provides a method of treating a disease in a subject comprising administering to the subject a DNA product described herein. The invention also provides a method of treating a disease in a subject comprising administering to the subject the DNA product produced by the methods described herein. The invention also provides a method of treating a disease in a subject comprising administering to the subject the DNA product obtainable by the methods described herein. Preferably, the amount of the DNA product administered to the subject is a therapeutic active amount.

The DNA product described herein may be used to treat any disease or disorder. For example, the DNA product may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the DNA product is used to treat a genetic disorder. More preferably still, the DNA product is used to treat a monogenic disorder. For example, the DNA product, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

A subject treated with the DNA product may receive the DNA product in the form of any of the pharmaceutical compositions described herein.

A subject treated with the DNA product may receive the DNA product in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the DNA product into the subject's body as described in more detail above (see "Methods for producing a pharmaceutical composition"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the DNA product that, when administered to a subject for treating a disease, is sufficient to affect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides the use of the DNA product as described herein in a method of diagnosing a disease and/or a disorder. The invention also provides the use of the DNA product produced or obtainable by the methods described herein in a method of diagnosing a disease and/or a disorder.

The invention provides the use of the DNA product in the "in vitro" diagnosis of a disease. The invention provides the use of the DNA product obtainable by the methods described herein in the "in vitro" diagnosis of a disease.

The invention also provides the DNA product for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the DNA product is used to diagnose a genetic disorder. More preferably still, the DNA product is used to diagnose a monogenic disorder. For example, the DNA product may be used to diagnose sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the DNA product.

To facilitate detection and/or quantification of the DNA product, the DNA product may be attached or bound to a functional portion. The functional portion may be any functional portion described herein. For example, the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the DNA product. For example, the signal may be measured by flow cytometry and/or fluorescence-activated cell sorting of a DNA product attached to a fluorescent probe.

The DNA product may be detected by being bound to a capture moiety, for example in a lateral flow assay. In this example, the functional portion is a protein, for example, an antibody specific for the capture moiety. The capture of the antibody attached to the DNA product may produce a visual signal (e.g. a band of a different colour).

The invention also provides a method that combines diagnosis of a disease with a treatment of the disease.

### Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the DNA product may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the DNA product described herein for use in cell therapy. The invention provides the DNA product produced or obtainable by the methods described herein for use in cell therapy.

The invention provides the DNA product described herein for use in cell therapy. The invention provides the DNA product produced or obtainable by the methods described herein for use in cell therapy.

Preferably, cell therapy is ex-vivo cell therapy. The cell may be an animal cell, preferably mammal cell, such as human cell.

The invention also provides a cell obtainable by any methods described herein. The cell may be suitable for use in cell therapy.

### Vaccines

The DNA products produced by the methods described herein are particularly suitable for use in vaccine production. A vaccine may comprise a DNA product described herein. A vaccine may comprise a DNA product produced or obtainable by the methods described herein. Alternatively, the DNA product may be used to produce a vaccine, preferably an mRNA-based vaccine. For example, vaccines such as the BioNTech and Moderna mRNA vaccines against severe acute respiratory syndrome coronavirus 2 (SARS-CoV-2). The DNA product may be used to produce a seasonal, pandemic, prophylactic or therapeutic vaccine.

Thus, the invention provides the use of the DNA product described herein in the production of a vaccine. The invention also provides the use of the DNA product produced or obtainable by the methods described herein in the production of a vaccine.

The DNA product may encode an antigen, which may cause an immune response in a subject. The subject may be human. Preferably, the antigen is encoded on the DNA cassette. The DNA product may encode a neoantigen, which may cause an immune response in a subject. Preferably, the neoantigen is encoded on the DNA cassette.

### CAR-T cells

The invention provides the use of a DNA product described herein in the production of a CAR-T cell. The invention provides the use of the DNA product produced or obtainable by the methods described herein in the production of a CAR-T cell.

The invention provides a method for producing a genetically engineered CAR-T cell comprising: (a) introducing the DNA product described herein into a T cell; and (b) expressing a gene of interest encoding by the DNA product. Preferably, the gene of interest is a tumour-specific CAR.

The invention provides a method for producing a genetically engineered CAR-T cell comprising: (a) introducing the DNA product obtainable by the methods described herein into a T cell; and (b) expressing a gene of interest encoding by the DNA product. Preferably, the gene of interest is a tumour-specific CAR.

The method may further comprise, before step (a) (i.e. introducing the DNA product into a T cell), a step of removing mononuclear cells from a patient. Preferably, the step of removing is performed using leukapheresis. Preferably the mononuclear cells are T cells. The method may further comprise, after step (b) (i.e. expressing a gene of interest), a step of returning the genetically engineered cells to the patient.

The invention also provides an engineered T cell obtainable by any methods described herein. The engineered T cell may be suitable for use in CAR T-cell therapy.

### CRISPR delivery

The products produced by the methods described herein are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The DNA product may comprise a gene sequence encoding any component of the CRIPSR machinery. The DNA product may encode all components of the CRIPSR machinery.

The DNA product may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cpf1 or Mad7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The DNA product encoding the repair template may be delivered to a cell by a nanoparticle, a non-viral vector or a viral vector, or without the aid of any carrier. The DNA product encoding the repair template may be delivered to a cell by electroporation. The DNA product encoding the repair template may be delivered to a cell by hydrodynamic needle.

The DNA product may comprise (or further comprise) a gene sequence encoding a nuclease protein of a CRISPR system (e.g. Cpf1 or Mad7), and/or a guide RNA. The DNA product may comprise (or further comprise) a gene sequence encoding a nuclease protein of a CRISPR system (e.g. Cpf1, or Mad7). The DNA product may comprise (or further comprise) a gene sequence encoding a guide RNA. The DNA product may comprise (or further comprise) a gene sequence encoding a nuclease protein of a CRISPR system (e.g. Cpf1, or Mad7) and a guide RNA. The DNA product may comprise (or further comprise) a gene sequence encoding a genomic target to be modified (e.g. a spacer). The DNA product may be ligated to a vector. The vector may comprise a sequence of some of the components of the CRIPSR system. The vector may comprise a sequence of guide RNA or a sequence of a part of the guide RNA. If the vector comprises a sequence of a part of the guide RNA, the DNA product may comprise the missing sequence part of the guide RNA, so that upon ligation, the ligated vector comprises a full sequence of a guide RNA. The nuclease of the CRISPR system and guide RNA may be encoded on a single vector or on two different vectors. The DNA product may encode the nuclease of the CRISPR system and guide RNA. One DNA product may encode the nuclease of the CRISPR system, and the other DNA product may encode guide RNA.

The DNA product may be for use in CRISPR-Cas mediated repair by recombination, homology-directed repair, or non-homologous end joining.

If the nuclease of the CRISPR system and the guide RNA are encoded by a different DNA product, they may be part of a different or the same delivery mechanism. For example, the DNA product encoding the nuclease of the CRISPR system may be delivered to a cell by a first nanoparticle, non-viral vector or viral vector, whereas the DNA product encoding the guide RNA may be delivered to a cell by a second nanoparticle, a non-viral vector, or viral vector. For example, the DNA product encoding the nuclease of the CRISPR system and the DNA product encoding the guide RNA may be delivered to a cell by the same nanoparticle, non-viral vector or viral vector.

If the nuclease of the CRISPR system and the guide RNA are encoded by the same DNA product (or by a vector that comprises the DNA product) they may be part of the same delivery mechanism. For example, the DNA product, or the vector, encoding the nuclease of the CRISPR system and the guide RNA may be delivered to a cell by a nanoparticle, a non-viral vector or a viral vector.

The DNA product encoding the nuclease of the CRISPR system and the DNA product encoding the guide RNA may be delivered to a cell by electroporation. The DNA product encoding the nuclease of the CRISPR system and the DNA product encoding the guide RNA may be delivered to a cell by hydrodynamic needle.

Thus, the invention provides the DNA product described herein for use in CRISPR system delivery to a cell. The invention provides a method of delivering the CRISPR system to a cell, comprising contacting the DNA product described herein with a cell. The invention also provides the DNA product obtainable by the methods described herein for use in CRISPR system delivery to a cell. The invention provides a method of delivering the CRISPR system to a cell, comprising contacting the DNA product obtainable by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

The DNA product produced or obtainable by the methods described herein may be used in transcription, to generate RNA, preferably mRNA, in vitro or in vivo.

Each aspect or embodiment as defined herein may be combined with any other aspect(s) or embodiment(s) unless clearly indicated to the contrary. In particular, any feature indicated as being preferred or advantageous may be combined with any other feature or features indicated as being preferred or advantageous.

The foregoing detailed description has been provided by way of explanation and illustration and is not intended to limit the scope of the appended claims. Many variations in the presently preferred embodiments illustrated herein will be apparent to one of ordinary skill in the art and remain within the scope of the appended claims and their equivalents.

### BRIEF DESCRIPTION OF THE FIGURES

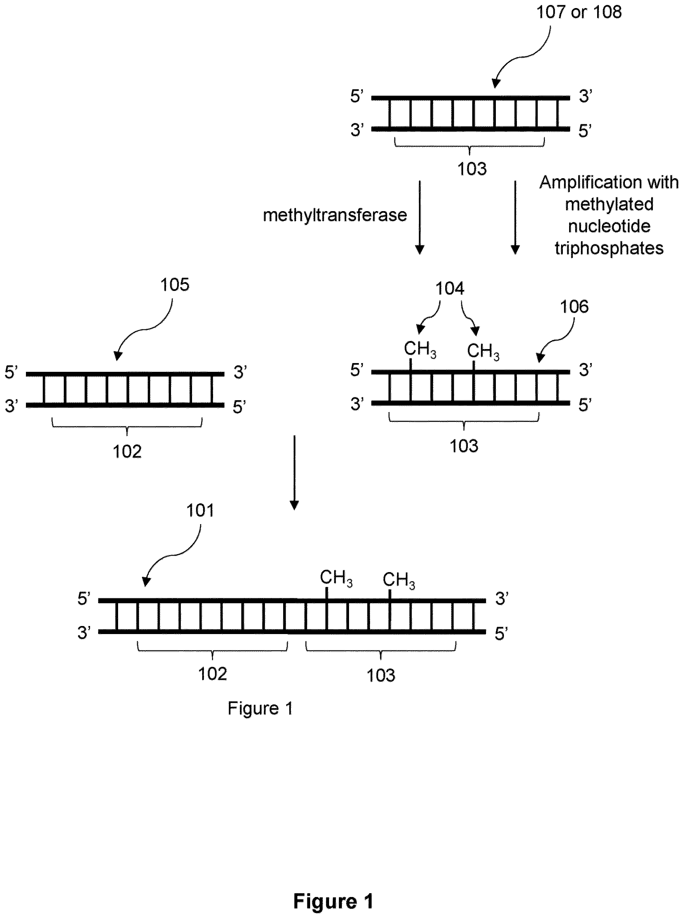

**FIG. 1** shows a method for producing a deoxyribonucleic acid (DNA) product (101) comprising an unmethylated region (102) and a methylated region (103), wherein the unmethylated region comprises a promoter sequence, wherein the methylated region comprises one or more methylated CpG sites (104), and wherein the method comprises:
   appending a first DNA molecule (105) comprising the unmethylated region to a second DNA molecule (106) comprising the methylated region to generate the DNA product.
   The second DNA molecule may be generated by amplifying a second DNA template molecule (108) in the presence of methylated nucleotide triphosphates to generate the second DNA molecule comprising the methylated region. The second DNA molecule may be generated by contacting a precursor DNA molecule (107) with a methyltransferase.
**FIG. 2** Illustrates the workflow used to obtain a compound DL product (two individual DNA sequences ligated together to form a single construct with adaptors ligated on either end of the construct using a single pot reaction).
**FIG. 3A** **and** **FIG. 3B** Agarose gel (0.8%) image showing the compound DL product from single pot compound DL (digestion and ligation) reaction and exonuclease treatments.
**FIG. 4A** **and** **FIG. 4B** Agarose gel (0.8%) image showing the compound DL product obtained after the compound DL reaction, Proteinase K and exonucleases treatments.

### EXAMPLES

### Example 1: Workflow to obtain the compound DL product

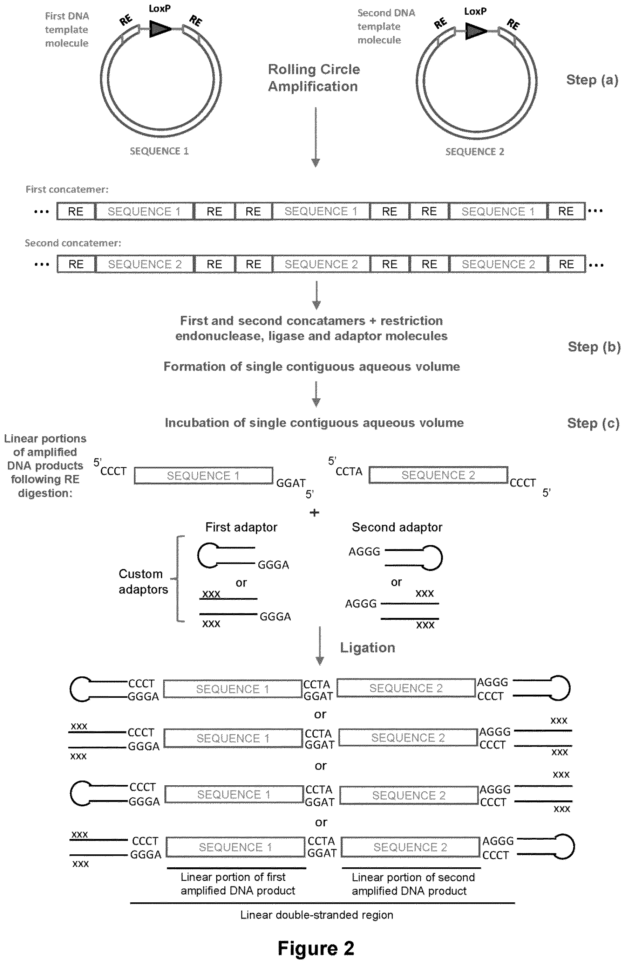

Rolling circle amplification (RCA) was carried out separately on both the first DNA template molecule (sequence 1) and the second DNA template molecule (sequence 2) (as shown in Step (a) of FIG. 2) to generate first and second DNA molecules, which are concatemers (long DNA molecules that contain the same copy of a DNA sequence linked in series with restriction endonuclease target sequences (denoted "RE") flanking each copy of the DNA sequence. As shown in FIG. 1, the second DNA molecule may be generated by amplifying a second DNA template molecule in the presence of methylated nucleotide triphosphates to generate the second DNA molecule comprising the methylated region. The first DNA molecule may be generated by amplifying a first DNA template molecule in the absence of methylated nucleotide triphosphates to generate the second DNA molecule comprising the unmethylated region.

The template DNA molecules were circularized prior to the RCA reaction so that they are compatible with the RCA reaction. The first and second concatemers were combined in single pot compound DL (Digestion and Ligation) reaction along with a restriction endonuclease, ligase, and first and second adaptor molecules (hairpin, or linear adaptor molecules comprising nuclease resistant nucleotides (denoted "xxx" in FIG 2)) in a reaction buffer (Step (b) of FIG. 2). A single pot reaction involves simultaneous digestion and ligation reactions whereby complementary overhangs are ligated after digestion by restriction enzymes or endonucleases which are specific to restriction or endonuclease sites flanking the sequences of interest. Concatemers produced via the RCA reactions (first and second concatamers) were digested by a restriction enzyme present in the compound DL reaction to produce first and second linear portions of the amplified DNA products with overhangs (as shown in Step c of FIG. 2). The downstream overhang of the linear portion of the first amplified DNA product is complementary only to the upstream overhang of the linear portion of the second amplified DNA product; in addition, first and second adaptor molecule overhangs are complementary only to the upstream and downstream of the linear portions of the first and second amplified DNA products, respectively. Complementary overhangs are ligated by ligases to produce the compound DL product (as shown in Step c of FIG. 2). The product obtained at the end of the compound DL reaction is referred to as the compound DL product and consists of a linear portion of the first amplified DNA product and a linear portion of the second amplified DNA product ligated together to form a single construct (the linear double-stranded region) with first and second adaptor molecules ligated on either end of the construct (as shown in Step c of FIG. 2). The products of the compound DL reaction may also contain off target ligation products or non-ligated products; therefore, the single pot reaction was subjected to exonuclease treatment to remove any off-target ligation products or non-ligated products that were present.

In addition, to potentially obtain just a band corresponding to the compound DL product with no trace of high molecular weight product in the well after agarose gel analysis, Proteinase K treatment was introduced in between the single pot digestion and ligation step and the exonuclease treatment step to see whether Proteinase K treatment enhances the performance of the overall process by reducing the enzyme burden that is carried on to the exonuclease treatment step.

### Example 2: Production of the compound DL product

Sequence 1 and sequence 2 were amplified in individual RCA reactions. Before the amplification, the DNA sequences were circularized, and then the circularized DNA sequences were first denatured by adding 1 volume of buffer D (400 mM KOH, 10 mM EDTA) and incubating for 3 min at room temperature. The samples were then neutralized by adding 1 volume of buffer N (400 mM HCl, 600 mM Tris-HCl pH 7.5). The RCA conditions were as follows: 1 ml reaction volume, 0.01 ml TruePrime Whole Genome Amplification (WGA) reaction buffer 10x (4basebio), 0.150 mL denatured DNA sample, 0.046 ml TthPrimPol (740 ng/uL), 0.016 mL QualiPhi Phi29DNApol (1000 ng/uL), 0.0025mL PPase (80 ng/uL) and 0.04 mL dNTPs (25 mM). Incubation time and temperature: 20 hours at 30°C, followed by incubation for 10 min at 65°C.

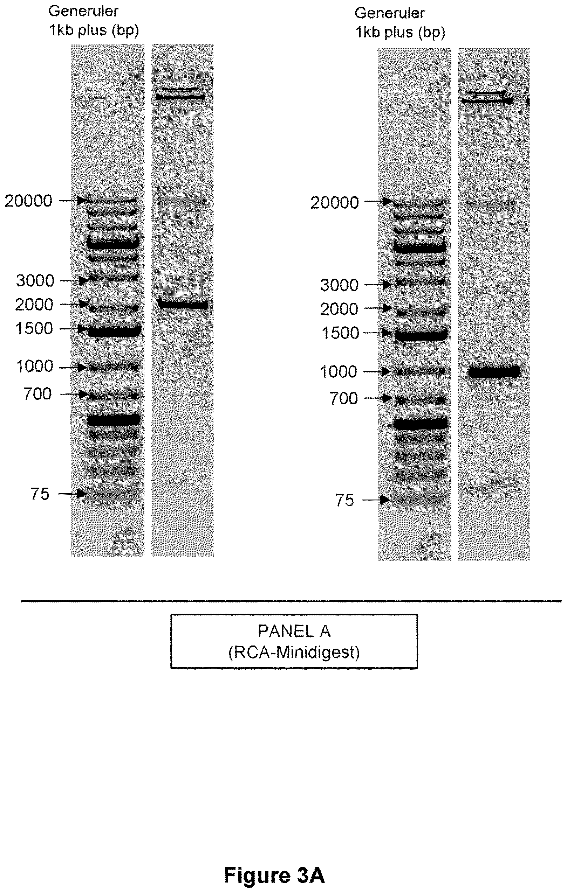

The RCA material aliquot was subjected to Bsal digestion before feeding the material into the compound DL reaction to confirm that the size of the amplified products for sequence 1 and sequence 2 were as expected. FIG. 3A, Panel A shows that upon Bsal digestion, sequence 1 produced a band of ~1000 bp and sequence 2 produced a band of -2000 bp, which was as expected. Following this confirmation the amplified sequences were further processed to produce the compound DL product.

In the compound DL reaction, equivolume of sequence 1 and sequence 2 with an initial concentration of 499.5ng/ul (obtained after amplifying the sequences individually in RCA reactions) were combined in a single pot compound DL reaction to give a final DNA concentration of 240ng/uL in a total volume of 0.2 ml. The components of the compound DL reaction consisted of RCA DNA material (240ng DNA/uL), enzymes (Bsal (3.2 ng/uL 4Basebio Bsal)), ligases (T3 Ligase NEB (4.95 U/uL) or T4 Ligase 4Basebio (8.3 ng/uL) or T7 Ligase NEB (4.95 U/uL)), hairpin adaptors (10x molar excess of adaptor per DNA end), digestion ligation reaction buffer (50 mM Tris HCl [pH 7.5], 10 mM MgCl2, 10 mM DTT) and 10mM ATP. The reaction was incubated for 18 h at 25°C or 37°C.

Sequence 1 is 927 bp, sequence 2 is 1968 bp. When ligated a 2895 bp construct was formed. The final size of the construct was used for calculations of 10x molar excess of the hairpin adaptor required per DNA end. The addition of hairpin adaptors protects the construct from exonuclease activity and any molecules not flanked by hairpin adaptors are digested by the exonucleases.

The digested material and hairpin adaptors were ligated using one of three ligase types: T3 ligase (with a final concentration of 4.95 U/uL), T4 ligase (with a final concentration of 8.3ng/uL) or T7 ligase (with a final concentration of 4.95 U/uL).

Aliquots were taken after the compound DL reaction and the remaining single pot compound DL reaction was further treated with exonuclease I (final concentration of 4.462 ng/uL) and exonuclease III (final concentration of 8.428 ng/uL) and incubated for 2 h at 37°C to digest non-ligated or off target product. After exonuclease treatment, further aliquots were taken for analysis.

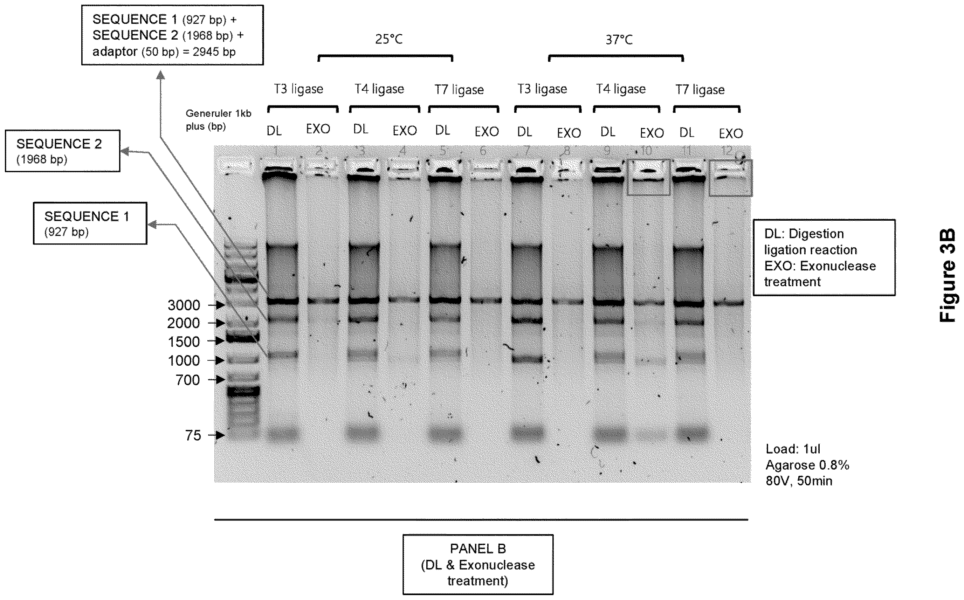

Fluorometer (Qubit) measurements were taken for each of the aliquots taken after the single pot compound DL reaction and after exonuclease treatment. All of the ligase conditions produced a compound DL product of -3000bp. Since the construct (sequence 1 + sequence 2) was flanked by hairpin adaptors, the construct withstood exonuclease treatment. Furthermore, with T7 ligase there was no off-target ligation product carry over after exonuclease treatment at both the 25°C and 37°C incubation temperatures (see FIG. 3B, Panel B, lane 6 and lane 12), whereas the T4 ligase-containing reaction showed carry over of the off-target ligation product of ~1000 bp (sequence 1) at the 25°C incubation temperature (see FIG. 3B, Panel B, lane 4) and the off target ligation products of ~ 1000bp (sequence 1) and ~2000bp at the 37°C incubation temperature (sequence 2) (see FIG. 3B, Panel B, lane 10) after exonuclease treatment.

On the other hand, carry over of the off target ligation product of -2000 bp (sequence 2) was observed for the T3 ligase condition at the 25°C incubation temperature (see FIG. 3B, Panel B, lane 2) but no off-target ligation product was observed at 37°C (see FIG. 3B, Panel B, lane 8) after exonuclease treatment.

**Table 2: Shows the DNA concentration measurement using Qubit and agarose gel densitometry analysis for compound DL, exonuclease treated product.**

| **Incubation temperature °C** | **Ligase type** | **After compound DL (ng/ul)** | **After exonuclease treatment (ng/ul)** | **Qubit ligation efficiency %** | **Agarose gel Densitometry ligation efficiency %** |
|---|---|---|---|---|---|
| 25°C | T3 ligase | 279 | 61.3 | 21.97 | 76.13 |
| | T4 ligase | 284 | 75.15 | 26.46 | 55.85 |
| | T7 ligase | 266 | 73.25 | 27.54 | 65.66 |
| 37°C | T3 ligase | 289 | 55.65 | 19.26 | 73.99 |
| | T4 ligase | 269 | 58.4 | 21.71 | 41.34 |
| | T7 ligase | 257 | 88.5 | 34.44 | 67.24 |

Even though T3 ligase has a higher ligation efficiency of 76.13 % compared to T7 ligase with a ligation efficiency of 65.66%, T7 ligase at 25°C worked the best compared to T3 or T4 ligase as the T7 ligase condition generated reliable data and obtained just the product of interest with no off-target ligation products. Although T3 ligase condition obtained just the product of interest, its Qubit value of 55.65 ng/ul was lower than the T7 ligase Qubit value of 88.5 ng/ul. Again, the agarose gel densitometry ligation efficiency for T7 ligase of 34.44% was higher than the T3 ligase efficiency of 19.26 %. Both the 25°C and 37°C incubation temperatures for the ligase condition data showed that T7 ligase works the best in terms of product yield and ligation efficiency despite changing the incubation temperatures by producing just the product of interest with no off-target ligation products.

### Example 3: Proteinase K treatment between compound DL reaction and exonuclease treatment

Proteinase K treatment was performed between the compound DL reaction and exonuclease treatment. This was based on the assumption that Proteinase K would reduce the enzyme burden carried from the compound DL reaction into the enzymatic purification step and result in a better performance of exonuclease enzymes, producing a purer compound DL product.

Individual rolling circle amplification reactions were performed for each circularised template containing sequence 1 or sequence 2. Before amplification, circularized DNA sequences were first denatured by adding 1 volume of buffer D (400 mM KOH, 10 mM EDTA) and incubating for 3 min at room temperature. Samples were then neutralized by adding 1 volume of buffer N (400 mM HCl, 600 mM Tris-HCl pH 7.5). The conditions for RCA were as follows: 1 ml reaction volume, 0.01 ml TruePrime WGA reaction buffer 10x (4basebio), 0.150 mL denatured DNA sample, 0.046 ml TthPrimPol (740 ng/uL), 0.016 mL QualiPhi Phi29DNApol (1000 ng/uL), 0.0025mL PPase (80 ng/uL) and 0.04 mL dNTPs (25 mM). Incubation time and temperature: 20 hours at 30°C and followed by incubation for 10 min at 65°C.

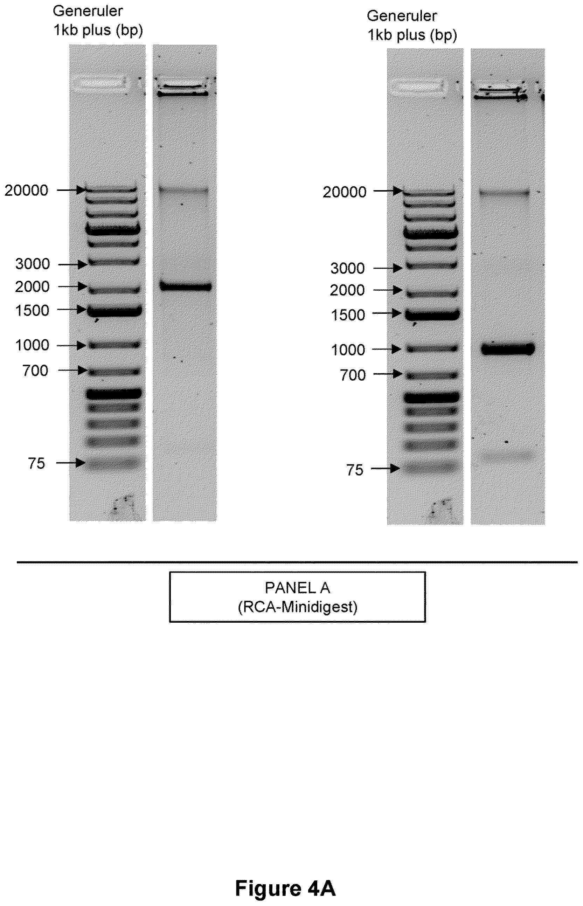

The aliquots of each RCA reaction material were subjected to Bsal digestion and evaluated for expected size in agarose gel analysis before feeding the material into a compound DL reaction. FIG. 4A, Panel A shows that upon Bsal digestion, a band of ~1000 bp (corresponding to sequence 1) and a band of and -2000 bp (corresponding to sequence 2) were produced, as expected. Upon confirmation these amplified sequences were further processed to produce the compound DL product.

In the compound DL reaction, equivolume of sequence 1 and sequence 2 with an initial concentration of 499.5ng/ul (obtained after amplifying them individually in RCA processes) were combined in a single pot compound DL reaction to give a final DNA concentration of 240ng/uL in total volume of 0.2 ml. The components of the compound DL reaction consisted of: RCA DNA material (240ng DNA/uL), enzymes (either Bsal or another endonuclease with a restriction site present in the RCA concatemer (3.2 ng/uL 4Basebio Bsal)), ligases (T4 Ligase 4Basebio (8.3 ng/uL) or T7 Ligase NEB (4.95 U/uL)), hairpin adaptors (usually 10x molar excess of adaptor per DNA end), digestion ligation reaction buffer (50 mM Tris HCl [pH 7.5], 10 mM MgCl2, 10 mM DTT) and 10mM ATP. The reaction was incubated for 18 h at 37°C.

Sequence 1 is 927 bp, sequence 2 is 1968 bp. When ligated, a 2895 bp construct was formed. This final size of the construct was used for calculations of 10x molar excess of the hairpin adaptor required per DNA end. The addition of hairpin adaptors protects the construct from exonuclease activity and any molecules not flanked by hairpin adaptors are digested by the exonucleases.

Digested material and hairpin adaptors were ligated using one of two ligase types: T4 ligase (with a final concentration of 8.3ng/uL) or T7 ligase (with a final concentration of 4.95 U/uL).

Aliquots were taken after the compound DL reaction and the remaining single pot compound DL reaction mixture was further treated with Proteinase K enzyme (0.4 ug/mL) at 55 °C for 1 h. After the incubation, Proteinase K inhibitor (0.25mM) was added to the reaction to inhibit the activity of Proteinase K and was incubated at room temperature for at least 15 min before the addition of exonuclease to the reaction.

Aliquots were taken after the Proteinase K treatment and rest of the reaction was further treated with exonuclease I (final concentration of 4.5 ng/uL) and exonuclease III (final concentration of 8.4 ng/uL) and incubated for 2 h at 37°C to digest non-ligated or off target product. After the exonuclease treatment, aliquots were taken for analysis.

Qubit measurements were taken for each aliquot after the single pot compound DL reaction and after exonuclease treatment.

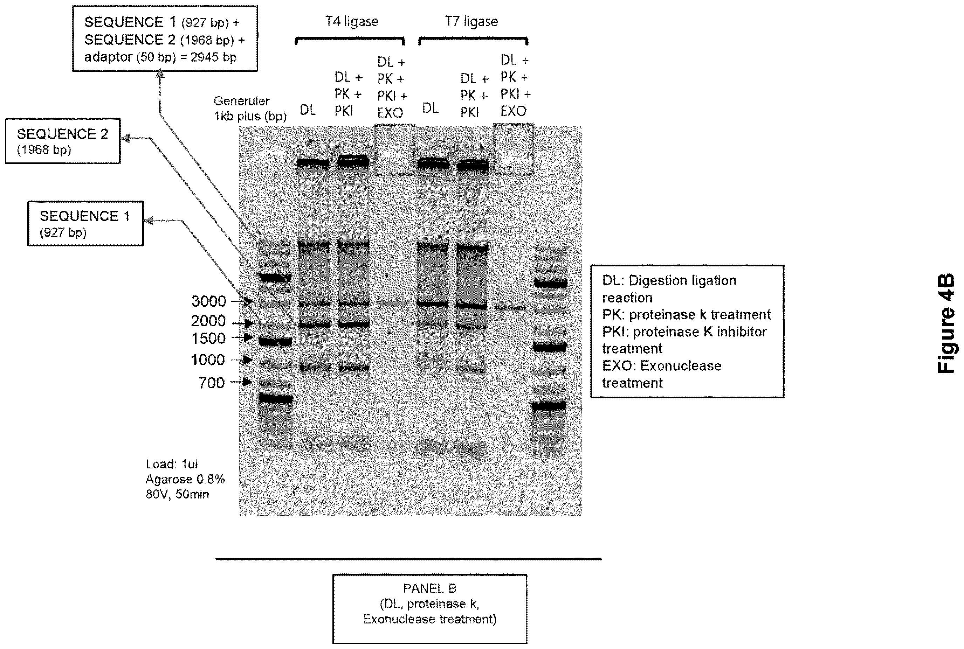

The outcome of the study showed that including Proteinase K treatment could improve the product quality. Both ligase conditions produced a compound DL product of -3000bp. Since the construct (sequence 1 + sequence 2) was flanked by hairpin adaptors, the construct withstood exonuclease treatment. Furthermore, T7 ligase had no off-target ligation product carry over after exonuclease treatment (see FIG. 4B, Panel B, lane 6), whereas the T4 ligase-containing reaction showed carry over of the off-target ligation product of ~1000 bp (corresponding to sequence 1) (see FIG. 4B, Panel B, lane 3) after exonuclease treatment.

The addition of the Proteinase K step between the compound DL and enzymatic purification steps (see FIG. 4B, Panel B, lane 3 and lane 6) as compared to the protocol without the Proteinase K step (see FIG. 4B, Panel B, boxes in lane 10 and lane 12) reduced the presence of high molecular weight amplified DNA and hence improved linear product purity. This result shows that Proteinase K treatment between the compound DL reaction and exonuclease treatment could be beneficial for producing a better quality compound DL product.

**Table 4: Shows the DNA concentration measurement using Qubit and agarose gel densitometry analysis for compound DL samples treated with Proteinase K before exonuclease treatment.**

| **Incubation temperature** | **Ligases** | **After compound DL (ng/ul)** | **After exonuclease treatment (ng/ul)** | **Qubit ligation efficiency %** | **Agarose gel Densitometry ligation efficiency %** |
|---|---|---|---|---|---|
| 37°C | T4 ligase | 268 | 27.4 | 10.46 | 65.25 |
| | T7 ligase | 260 | 30.7 | 11.3 | 72.42 |

Even though a major difference between T4 and T7 ligase Qubit values and ligation efficiency was not observed, the T7 Ligase condition worked better than theT4 ligase condition as the T7 ligase condition generated reliable data with just the product of interest and no off-target ligation products with a qubit value of 30.7 ng/ul and ligation efficiency of 72.42%

## Claims

1. A method for producing a deoxyribonucleic acid (DNA) product comprising an unmethylated region and a methylated region, wherein the unmethylated region comprises a promoter sequence, wherein the methylated region comprises one or more methylated CpG sites, and wherein the method comprises:
appending a first DNA molecule comprising the unmethylated region to a second DNA molecule comprising the methylated region to generate the DNA product.

2. The method of claim 1, wherein the method comprises amplifying a first DNA template molecule to generate the first DNA molecule comprising the unmethylated region.

3. The method of claim 1 or claim 2, wherein the method comprises amplifying a second DNA template molecule in the presence of methylated nucleotide triphosphates to generate the second DNA molecule comprising the methylated region.

4. The method of claim 1 or claim 2, wherein the method comprises contacting a precursor DNA molecule with a methyltransferase to generate the second DNA molecule comprising the methylated region; optionally wherein the precursor DNA molecule is substantially free from methylated nucleotides.

5. The method of claim 4, wherein the method comprises amplifying a second DNA template molecule to generate the precursor DNA molecule.

6. The method of claim 4 or 5, wherein the method comprises inactivating or removing the methyltransferase prior to appending the first DNA molecule to the second DNA molecule.

7. The method of any one of claims 1 to 6, wherein the DNA product is linear and optionally the method comprises:
(a) appending a first adaptor molecule to a first end of the DNA product and appending a second adaptor molecule to a second end of the DNA product, wherein the first adaptor molecule comprises a hairpin and/or one or more nuclease resistant nucleotides and the second adaptor molecule comprises a hairpin and/or one or more nuclease resistant nucleotides; or
(b) covalently closing one or both ends of the DNA product.

8. The method of any one of claims 1 to 7, wherein appending the first DNA molecule to the second DNA molecule comprises:
(a) contacting the first DNA molecule with an endonuclease, a ligase and the second DNA molecule to form a single contiguous aqueous volume; and
(b) incubating the single contiguous aqueous volume to generate the DNA product.

9. A deoxyribonucleic acid (DNA) product comprising an unmethylated region and a methylated region, wherein the unmethylated region comprises a promoter sequence, and wherein the methylated region comprises one or more methylated CpG sites.

10. The method of any one of claims 1 to 8 or DNA product of claim 9, wherein:
(a) the unmethylated region is 5' to the methylated region in the DNA product;
(b) the unmethylated region comprises CpG sites;
(c) the unmethylated region is not methylated on substantially all CpG sites;
(c) the unmethylated region comprises complementary sense and antisense strands;
(d) the methylated region comprises a coding sequence encoding a gene of interest;
(f) the methylated region is methylated on substantially all CpG sites;
(g) the methylated region comprises complementary sense and antisense strands; and/or
(h) the DNA product is linear and optionally comprises a closed loop and/or one or more nuclease-resistant nucleotides at one or both ends.

11. A kit comprising:
(A)
(a) a polymerase
(b) methylated nucleotide triphosphates; and
(c) a ligase; or
(B)
(a) a methyltransferase; and
(b) a ligase;
optionally wherein the kit further comprises an endonuclease and/or a first DNA template molecule comprising an unmethylated region comprising a promoter sequence.

12. A pharmaceutical composition comprising a DNA product and a pharmaceutically acceptable carrier or excipient, wherein the DNA product is the DNA product according to claim 9 or claim 10, or wherein the DNA product is obtainable or produced by the method of any one of claims 1-8 or 10.

13. A DNA product for use in therapy, wherein the DNA product is the DNA product according to claim 9 or claim 10, or wherein the DNA product is obtainable or produced by the method of any one of claims 1-8 or 10.

14. A vaccine comprising a DNA product, wherein the DNA product is the DNA product according to claim 9 or 10, or wherein the DNA product is obtainable or produced by the method of any one of claims 1-8 or 10.

15. A method for *in vitro* transcription of a DNA product, the method comprising:
(a) providing a DNA product, wherein the DNA product is the DNA product according to claim 9 or 10, or wherein the DNA product is obtainable or produced by the method of any one of claims 1-8 or 10;
(b) contacting the DNA product with a polymerase; and
(c) producing a transcription product.
